# EUROPEAN PATENT APPLICATION

(11) **EP 3 064 205 A1**
(43) Date of publication of application: **07.09.2016**
(21) Application number: 15158060.2
(22) Date of filing: 06.03.2015
(51) Int. Cl.: A61K 31/47, A61P 35/04

(54) **Therapeutic use of isomeric forms of 2-(4-chlorophenyl)quinolin-4-yl)(piperidin-2-yl)methanol**

(71) Applicant: Glionova AB, 111 53 Stockholm (SE)
(72) Inventor: Enfors, Patrik, 111 53 STOCKHOLM (SE); Hammarström, Lars, 111 53 STOCKHOLM (SE)
(74) Representative: Awapatent AB

(57) **Abstract**

The present invention relates to certain isomeric forms of the 2,4-disubstituted quinoline derivative Vacquinol-1 (NSC13316, (2-(4-chlorophenyl)quinolin-4-yl)(piperidin-2-yl)methanol), for use the in treatment of systemic cancer, as well as pharmaceutical compositions comprising said isomeric forms of the 2,4-disubstituted quinoline derivative Vacquinol-1 for the intended use.

## Description

### Field of the invention

The present invention relates to certain stereoisomeric forms of the 2,4-disubstituted quinoline derivative Vacquinol-1 (NSC13316, (2-(4-chlorophenyl)quinolin-4-yl)(piperidin-2-yl)methanol), for use the in treatment of systemic cancer, as well as pharmaceutical compositions comprising said stereoisomeric forms of the 2,4-disubstituted quinoline derivative Vacquinol-1 for the intended use.

### Background of the invention

Cancers are characterized by complex mutational profiles in key regulatory pathways ensuring sustained survival, proliferation, metastasis, deregulated metabolism and genomic instability, etc, described as the "Hallmarks of Cancer" (Hanahan, D., & Weinberg, R. A. (2000) Cell, 100(1), 57-70; Hanahan, D., & Weinberg, R. A. (2011) Cell, 144(5), 646-74). These disease characteristics are driven by inter- and intratumoral genetic and epigenetic variations, resulting in highly complex sub-types which are difficult to catagorize and target therapeutically (Gerdes, M. J., et al. (2014) Frontiers in Oncology, 4, 1-12).

In addition to driving oncogenesis, the complex mutational landscape of tumors and their abnormal microenvironment may also lead to unique acquired functions in cancer cells which are absent in normal tissues. In a screen of the NCI Diversity Set II from the National Cancer Institutet (NCI), Kitambi and co-workers (Kitambi, S. S., et. Al. (2014) Cell, 157(2), 313-328) identified the racemic quinoline methanol Vacquinol-1 (NSC13316) as a potent inducer of a novel cell death mechanism in glioma cells, namely catastrophic vacuolization which is not present in normal tissue and thus selective for cancer cells. The identifier "NSC13316" is a depositor-supplied synonym for (2-(4-chlorophenyl)quinolin-4-yl)(piperidin-2-yl)methanol which has the following chemical structure:

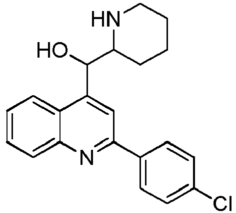

Vacquinol-1 readily crossed the blood-brain barrier (BBB) upon oral administration and dramatically extended survival in a murine orthotopic PDX model of human glioblastoma. Treatment of cancers requires exposure to the target organs where the tumors are present. In addition, in order to minimize side effects of treatment, it is desirable to minimize exposure of the chemotherapeutic agent to normal tissue. As Vacquinol-1 contains a mixture of four possible stereoisomers, it is possible that the distribution of these isomers may be different towards different target tissues and have different distributions in terms of systemic and CNS exposure.

### Summary of the invention

The present invention relates to providing certain stereoisomeric forms of the 2,4-disubstituted quinoline derivative Vacquinol-1 (NSC13316, (2-(4-chlorophenyl)quinolin-4-yl)(piperidin-2-yl)methanol) with favorable systemic exposure profiles, capable of selectively killing tumor cells and/or cancer stem cells with minimal effects on other cell types of the body.

More specifically, the invention relates to the use of certain stereoisomeric forms of 2,4-disubstituted quinoline derivatives in the treatment of systemic cancers, such as, but not limited to, pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/ lymphoid, pleura mesothelioma, renal, sarcoma, soft tissue, testicular, uterine body cancer.

In one aspect of the invention, there is provided (S)-(2-(4-chlorophenyl)quinolin-4-yl)((R)-piperidin-2-yl)methanol **(S2)** for use in the treatment of systemic cancer.

Another aspect of the invention features synthesis of the specific stereoisomers (R)-(2-(4-chlorophenyl)quinolin-4-yl)((S)-piperidin-2-yl)methanol **(S1)** and (S)-(2-(4-chlorophenyl)quinolin-4-yl)((R)-piperidin-2-yl)methanol **(S2).**

Another aspect of the invention features compositions (eg pharmaceutical compositions and medicaments) that include any one, two, three or four of the four possible stereosiomers of (2-(4-chlorophenyl)quinolin-4-yl)(piperidin-2-yl)methanol, or a pharmaceutically acceptable salt thereof.

Other aspects of the invention feature methods of treating systemic cancer, comprising administering to a subject a therapeutically effective amount of (S)-(2-(4-chlorophenyl)quinolin-4-yl)((R)-piperidin-2-yl)methanol **(S2),** pharmaceutical compositions comprising a mixture of (S)-(2-(4-chlorophenyl)quinolin-4-yl)((R)-piperidin-2-yl)methanol **(S1),** or a pharmaceutically acceptable salt or solvate thereof; and (R)-(2-(4-chlorophenyl)quinolin-4-yl)((S)-piperidin-2-yl)methanol **(S1)** and in particular compositions wherein said mixture comprises greater than 90% of (S)-(2-(4-chlorophenyl)quinolin-4-yl)((R)-piperidin-2-yl)methanol **(S2).**

### Brief description of the drawings

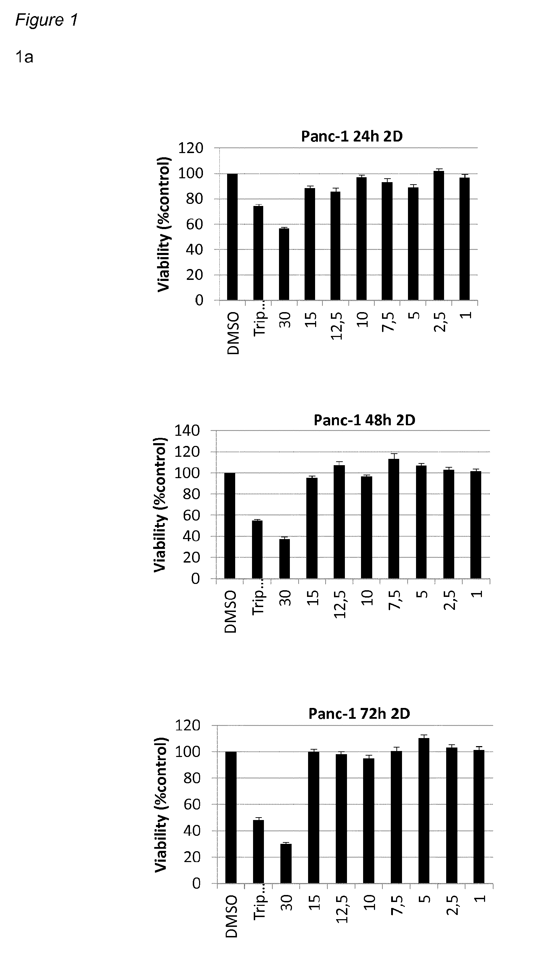

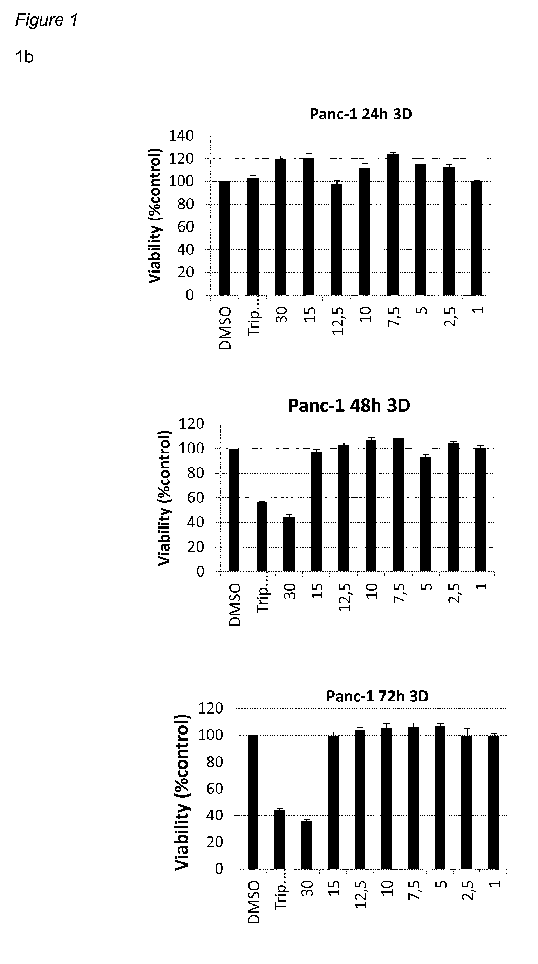

Figure 1 shows that treatment with Vacquinol-1 RS **(S1)** in Panc-1 cells in 2D culture, leads to loss of viability at 30µM in a time course manner; (after 24 h, 48h and 72h a decrease of 43%, 63% and 70 % in cell viability was evident). Treatment with Vacquinol-1 RS **(S1)** in Panc-1 cells in 3D culture, did not affect cells after 24h, however, after 48h and 72h a decrease of 55% and 64% in cell viability, was evident. At this concentration vacuoles were formed. Triptolide was used as positive control at (40 µM). X axis treatment with Vacquinol-1 RS **(S1)** at indicated concentrations in micromolar, DMSO or Triptolide.

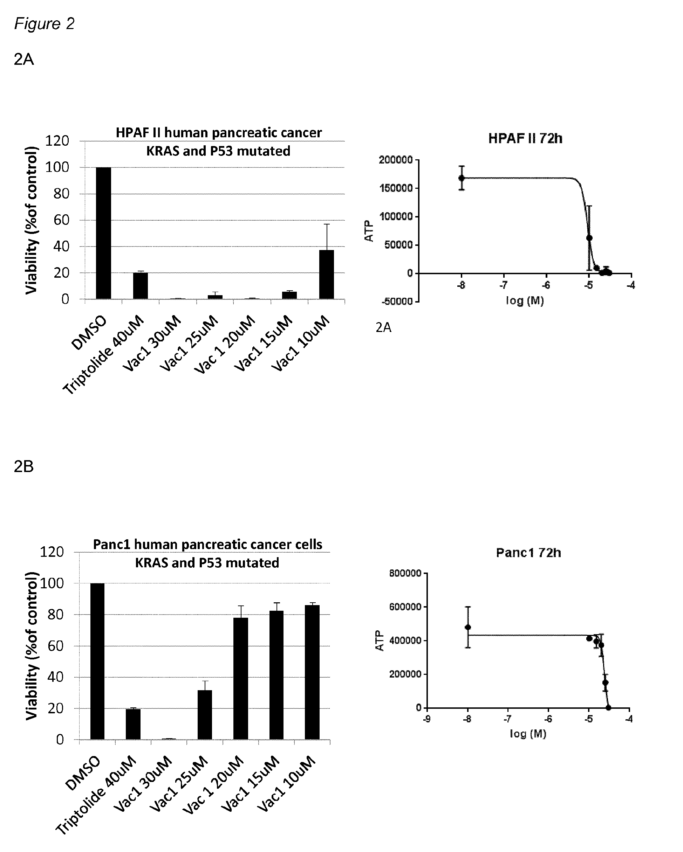

Figure 2 shows the effect of Vacquinol-1 RS **(S1)** on HPAF II and Panc-1 3D culture cell viability in the ATP viability assay at 72 h. HPAF II cells, which exhibit a mutation both in KRAS and P53 genes, showed a marked decrease in viability at 30-10 µM, compared to control. Panc-1 cells, which exhibit a mutation in the Kras and P53 gene showed a marked decrease in viability at 30 and 25 µM and ∼20% decrease in viability at lower concentrations (20-10 µM), compared to control.

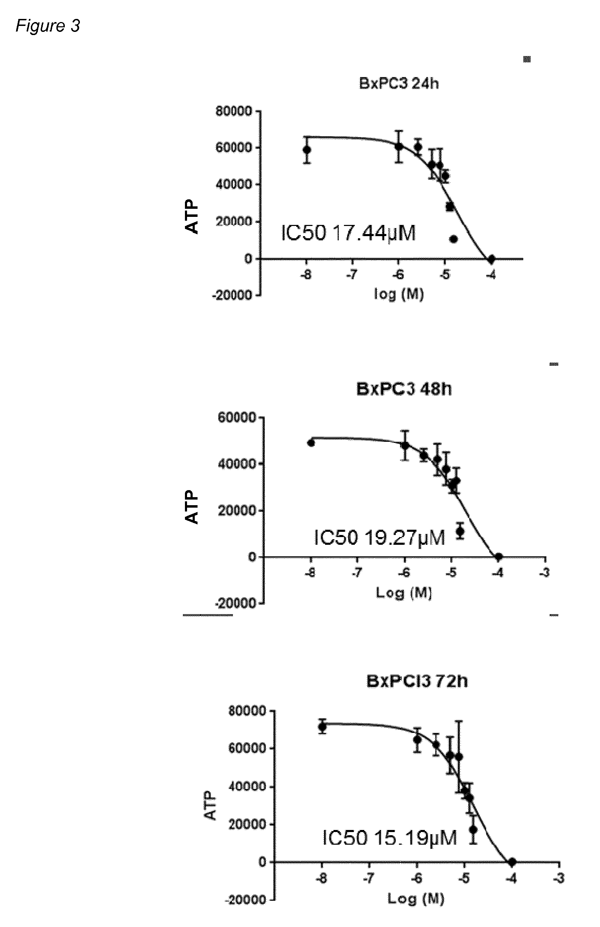

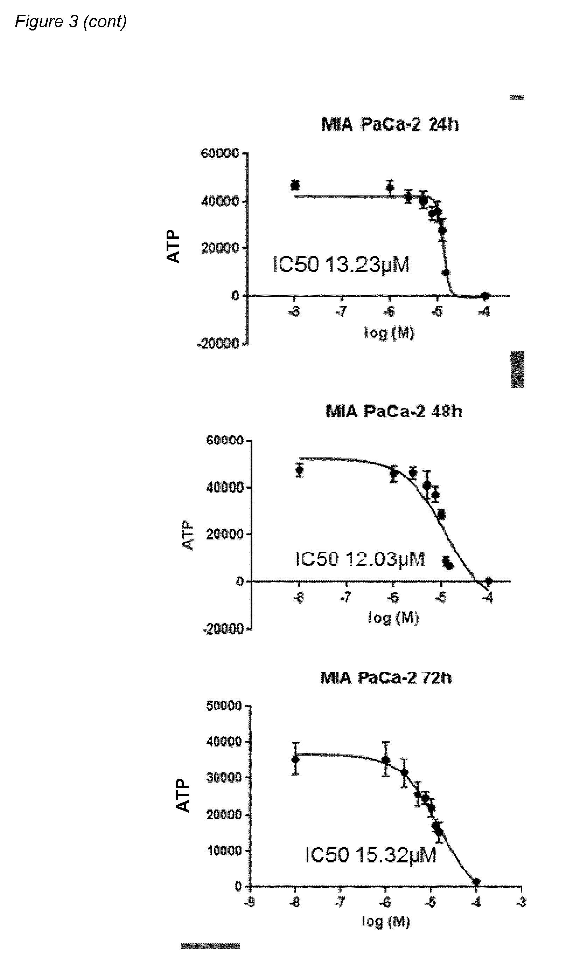

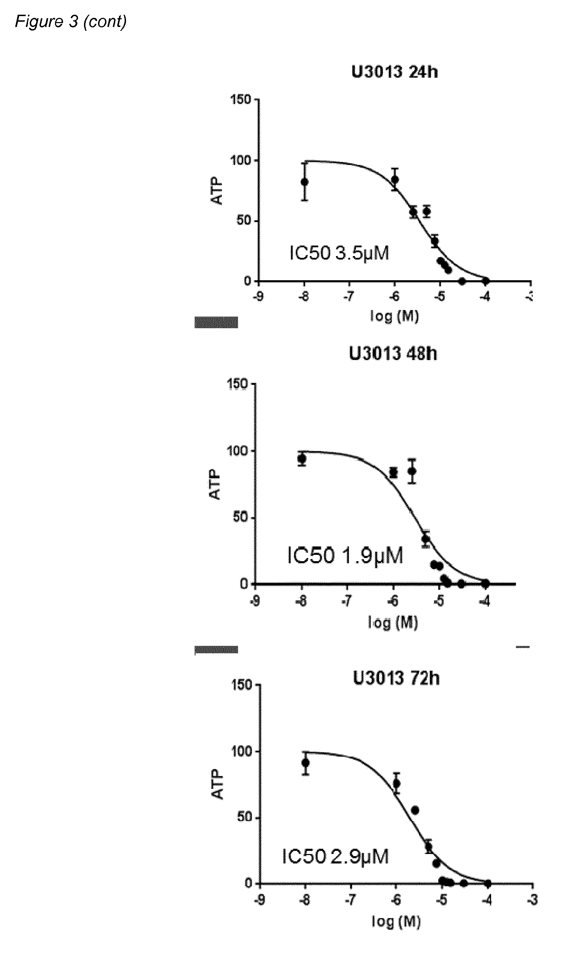

Figure 3 shows the effect on ATP viability assay of Vacquinol-1 RS **(S1)** on pancreatic BxPCI-3, MIA PaCa2 and U3013 at 25, 48 and 72 h of treatment. MIA-PaCa-2 cell line which carries RAS mutation, and BxPCI-2 cell line which exhibit wild type RAS were also tested for the effect of Vacquinol-1 RS **(S1)** on their viability, as measured by ATP viability assay. Both MIA-PaCa-2 cells which carries RAS mutation, and BxPCI-2 cells which exhibit wild type RAS show an IC50 ranging between 12-19 µM at 24h, 48h and 72 h, supporting an effect of Vacquinol-1 RS **(S1)** on viability of pancreatic adenocarcinoma cell lines, at a simlar range of Vacquinol concentrations. It also suggests that RAS does not play an important role in Vacquinol-1 RS (**S1**)-mediated cell death in these cells, however does not exclude other downstream targets. The glioblastoma U3013 cell line was tested in parallel as positive control for the effect of Vacquinol-1 RS **(S1)** on cell viability. The IC₅₀ ranged 3.5-1.9 µM.

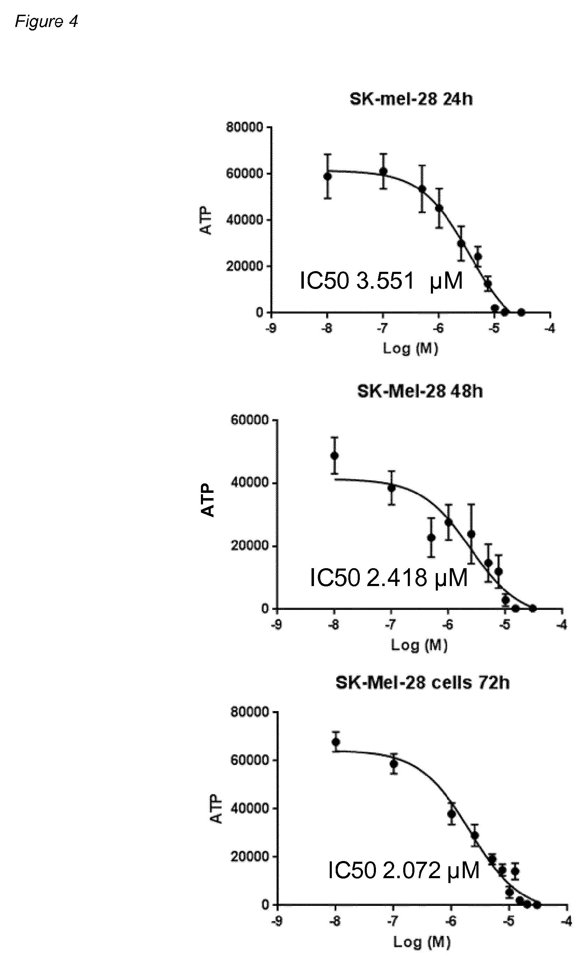

Figure 4 shows the effect of Vacquinol-1 SR **(S2)** on human melanoma SK-Mel-28 cell ATP viability assay at 24, 48 and 72 h of treatment. Vacquinol-1 SR **(S2)** treatment resulted in IC50 of 3.6, 2.4 and 2.1 µM, respectively, suggesting a high potency of Vacquinol-1 SR **(S2)** on melanoma cells.

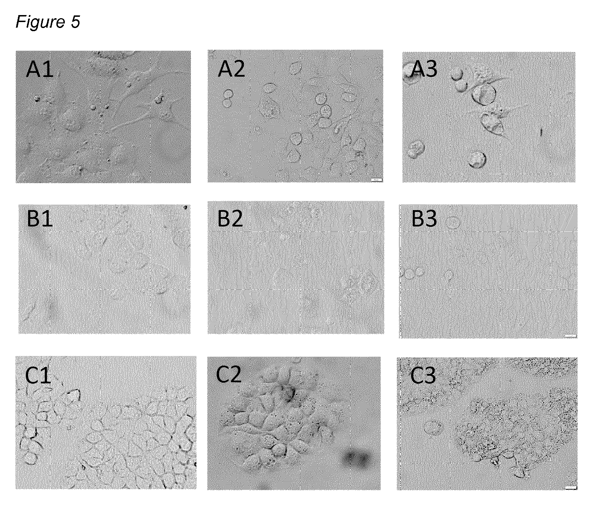

Figure 5 shows that the Vacquinol-1 SR **(S2)** stimulates vacuole formation. Bright field images of cells treated as follows. A1: SK-Mel-28 cells treated with 100% DMSO. A2: SK-Mel-28 cells treated with 5µM Vacquinol-1 SR for 18h. A3: SK-Mel-28 cells treated with 10µM S2 for 18h. B1: Panc-1 cells treated with 100 % DMSO B2: Panc-1 cells treated with 5µM Vacquinol-1 SR **(S2)** for 18h. B3: Panc-1 cells treated with 10µM S2 for 18h. C1: KPC cells were treated with 100% DMSO. C2: KPC cells were treated with 3.3 µM Vacquinol-1 SR **(S2)** for 18h. C3: KPC cells were treated with 10µM S2 for 18h. Note the lack of vacuoles in control cells (DMSO) compared to the Vacquinol-1 SR **(S2)**-treated cells in the indicated concentrations.

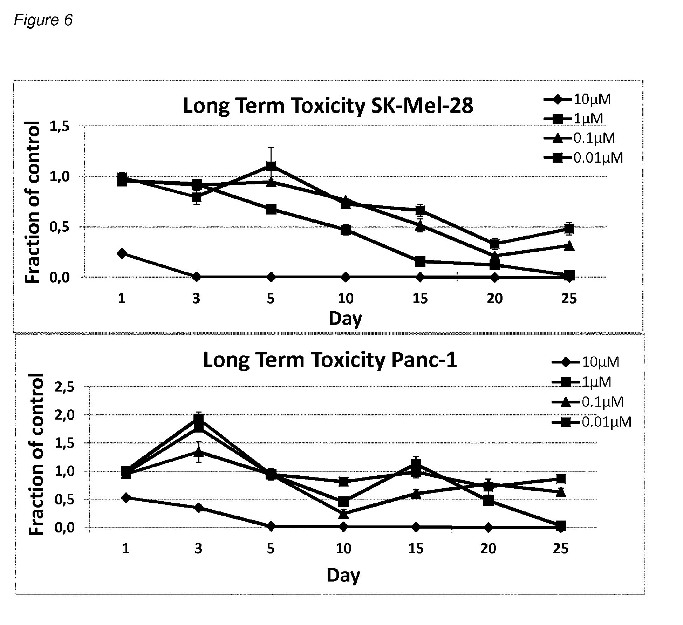

Figure 6 shows long term toxicity of Vacquinol-1 SR **(S2)** in Panc1 (top) and SK-Mel-28 cells (bottom). Vacquinol-1 SR **(S2)** exhibits a dose-dependent cytotoxic effect on Panc-1 cells. Note that long term exposure of lower concentrations (1µM) results in complete death. Fraction of control measured at all time-points. Vacquinol-1 SR **(S2)** exhibits a dose-dependent cytotoxic effect also on SK-Mel-28 cells. Note that long term exposure of lower concentrations result in complete death of the cells. Fraction of control measured at all time-points.

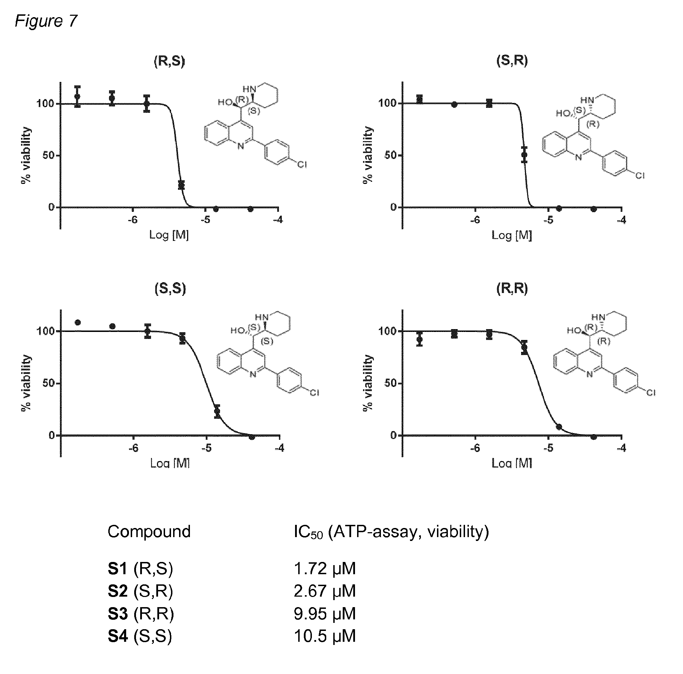

Figure 7 shows dose response evaluation of *in vitro* cytotoxicity of U3013 glioblastoma cells for the isolated isomers **(S1, S2, S3** and **S4)** of Vacquinol-1 as assessed by CellTiterGlo (Promega) ATP quantification. Compound **S1** and **S2** exhibit higher cytotoxicity on cancer cells than **S3** and **S4.** Each curve is generated based on quadruplicate replicates of each concentration/data point.

### Detailed description of the invention

The foregoing and other aspects of the present invention will now be described in more detail with respect to the description and methodologies provided herein. It should be appreciated that the invention may be embodied in different forms and should not be construed as limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the embodiments of the invention, the singular forms "a," "an" and "the" are intended to include the plural forms as well, unless the context clearly indicates otherwise. Also, as used herein, "and/or" refers to and encompasses any and all possible combinations of one or more of the associated listed items. Furthermore, the term "about," as used herein when referring to a measurable value such as an amount of a compound, dose, time, temperature, and the like, is meant to encompass variations of 20%, 10%, 5%, 1%, 0.5%, or even 0.1 % of the specified amount. When a range is employed *(e.g.,* a range from x to y) it is it meant that the measurable value is a range from about x to about y, or any range therein, such as about x₁ to about y₁, etc. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms, including technical and scientific terms used in the description, have the same meaning as commonly understood by one of ordinary skill in the art to which this invention belongs.

Anti-cancer activity of the racemic compound Vacquinol-1 (NSC13316) has been shown in glioma cells (Kitambi, SS et al, Cell, 157(2), 313-28) and therefore it was hypothesized therein that racemic Vacquinol-1 could be useful in treatment of non-systemic CNS-related types of cancer, such as glioblastoma.

Racemic Vacquinol-1 contains a mixture of 4 stereoisomers, namely:
(R)-(2-(4-chlorophenyl)quinolin-4-yl)((S)-piperidin-2-yl)methanol **(S1);**
(S)-(2-(4-chlorophenyl)quinolin-4-yl)((R)-piperidin-2-yl)methanol **(S2);**
(R)-(2-(4-chlorophenyl)quinolin-4-yl)((R)-piperidin-2-yl)methanol **(S3);** and
(S)-(2-(4-chlorophenyl)quinolin-4-yl)((S)-piperidin-2-yl)methanol **(S4).** The structures of **S1, S2, S3** and **S4,** respectively, are shown in the Scheme 1 below.

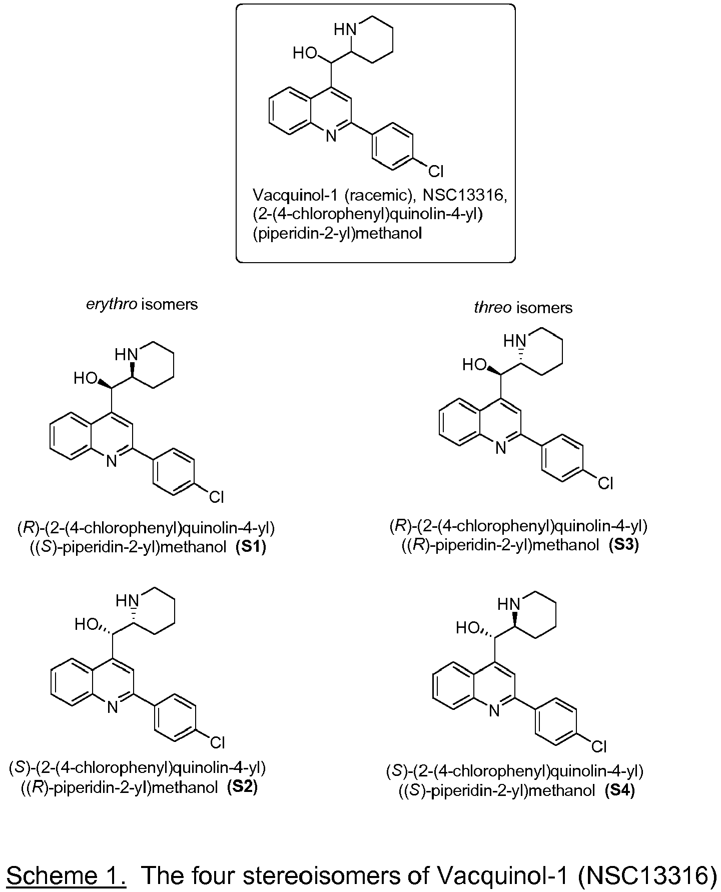

Any chiral center in a compound of the invention having a specified configuration is indicated as *R* or *S* using the well-known Cahn-Ingold-Prelog priority rules. Also, in any structural formula a chiral center having a specified configuration, (i.e. R or S) may be indicated using 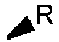 to indicate that the bond to R is directed out of the paper and towards the reader, and 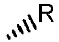 to indicate that the bond to R is directed out of the paper and away from the reader.

It was shown that the *erythro* stereoisomers **S1** and **S2** exhibited higher oncolytic activity than the corresponding *threo* stereoisomers **S3** and **S4.** It was further shown that between the *erythro* stereoisomers of Vacquinol-1, specifically (R)-(2-(4-chlorophenyl)quinolin-4-yl)((S)-piperidin-2-yl)methanol **(S1)** and (S)-(2-(4-chlorophenyl)quinolin-4-yl)((R)-piperidin-2-yl)methanol **(S2),** showed different *in vivo* pharmacokinetic properties and that compound **S1** showed preferential distribution into the central nervous system. In contrast, compound **S2** had considerably lower exposure in the central nervous system relative to its exposure in CNS tissue. Thus, use of compound **S2** in the treatment of periferal cancers such as pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer, would be advantageous as exposure to the central nervous system would be minimized and the risk of CNS-associated side effects minimized.

As used herein, a "compound" refers to the compound itself, including stereoisomers and tautomers thereof, and its pharmaceutically acceptable salts, solvates, hydrates, complexes, esters, prodrugs and/or salts of prodrugs, unless otherwise specified within the specific text for that compound. Except, when otherwise indicated, e.g. by indication of (R) or (S) configuration at a given location, all stereoisomers of the compounds of the instant invention are contemplated, either in admixture or in pure or substantially pure form. Consequently, compounds of the invention may exist in enantiomeric or racemic or diastereomeric forms or as mixtures thereof. The processes for preparation can utilize racemates or enantiomers as starting materials. When racemic and diastereomeric products are prepared, they can be separated by conventional methods, which for example are chromatographic or fractional crystallization.

The term "solvate" refers to a complex of variable stoichiometry formed e.g. by a compound of formula (I) and a solvent. The solvent is a pharmaceutically acceptable solvent, such as water, which should not interfere with the biological activity of the solute.

In one aspect of the invention, there is provided (S)-(2-(4-chlorophenyl)quinolin-4-yl)((R)-piperidin-2-yl)methanol **(S2),** or a pharmaceutically acceptable salt or solvate thereof, for use in the treatment of systemic cancer.

In one embodiment of this aspect, there is provided (S)-(2-(4-chlorophenyl)quinolin-4-yl)((R)-piperidin-2-yl)methanol, for use in the treatment of systemic cancer, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, there is provided (S)-(2-(4-chlorophenyl)quinolin-4-yl)((R)-piperidin-2-yl)methanol, for use in the treatment of pancreatic cancer.

In one embodiment of this aspect, there is provided (S)-(2-(4-chlorophenyl)quinolin-4-yl)((R)-piperidin-2-yl)methanol, for use in the treatment of skin cancer.

In one embodiment of this aspect, there is provided (S)-(2-(4-chlorophenyl)quinolin-4-yl)((R)-piperidin-2-yl)methanol, for use in the treatment of systemic cancer, wherein side effects on the central nervous system are minimized.

In another aspect of the invention, there is provided (R)-(2-(4-chlorophenyl)quinolin-4-yl)((S)-piperidin-2-yl)methanol, or a pharmaceutically acceptable salt or solvate thereof, for use in the treatment of systemic cancer.

In one embodiment of this aspect, there is provided (R)-(2-(4-chlorophenyl)quinolin-4-yl)((S)-piperidin-2-yl)methanol for use in the treatment of systemic cancer, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, there is provided (R)-(2-(4-chlorophenyl)quinolin-4-yl)((S)-piperidin-2-yl)methanol, for use in the treatment of systemic cancer, wherein said systemic cancer is pancreatic cancer.

In one embodiment of this aspect, there is provided (R)-(2-(4-chlorophenyl)quinolin-4-yl)((S)-piperidin-2-yl)methanol, for use in the treatment of systemic cancer, wherein said systemic cancer is skin cancer.

In another aspect of the invention, there is provided a method of treating systemic cancer, comprising administering to a subject a therapeutically effective amount of (S)-(2-(4-chlorophenyl)quinolin-4-yl)((R)-piperidin-2-yl)methanol or a pharmaceutically acceptable salt or solvate thereof.

In one embodiment of this aspect, said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, said systemic cancer is pancreatic cancer.

In one embodiment of this aspect, said systemic cancer is skin cancer.

In one embodiment of this aspect, side effects on central nervous system are minimized.

In another aspect of the invention, there is provided a method of treating systemic cancer, comprising administering to a subject a therapeutically effective amount of (R)-(2-(4-chlorophenyl)quinolin-4-yl)((S)-piperidin-2-yl)methanol or a pharmaceutically acceptable salt or solvate thereof.

In one embodiment of this aspect, said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, said systemic cancer is pancreatic cancer.

In one embodiment of this aspect, said systemic cancer is skin cancer.

In one embodiment of this aspect, side effects on central nervous system are minimized.

In another aspect of the invention, there is provided use of (S)-(2-(4-chlorophenyl)quinolin-4-yl)((R)-piperidin-2-yl)methanol or a pharmaceutically acceptable salt or solvate thereof, in the manufacture of a medicament for treatment of systemic cancer.

In one embodiment of this aspect, said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, said systemic cancer is pancreatic cancer.

In one embodiment of this aspect, said systemic cancer is skin cancer.

In one embodiment of this aspect, side effects on central nervous system are minimized.

In another aspect of the invention, there is provided use of (R)-(2-(4-chlorophenyl)quinolin-4-yl)((S)-piperidin-2-yl)methanol or a pharmaceutically acceptable salt or solvate thereof, in the manufacture of a medicament for treatment of systemic cancer.

In one embodiment of this aspect, said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, said systemic cancer is pancreatic cancer.

In one embodiment of this aspect, said systemic cancer is skin cancer.

In one embodiment of this aspect, side effects on central nervous system are minimized.

In another aspect of the invention, there is provided a phamaceutical composition comprising a mixture of (S)-(2-(4-chlorophenyl)quinolin-4-yl)((R)-piperidin-2-yl)methanol, or a pharmaceutically acceptable salt or solvate thereof; and (R)-(2-(4-chlorophenyl)quinolin-4-yl)((S)-piperidin-2-yl)methanol, or a pharmaceutically acceptable salt or solvate thereof, together with pharmaceutically acceptable excipients and diluents, for use in the treatment of systemic cancer.

In one embodiment of this aspect, said mixture comprises at least 90% of (S)-(2-(4-chlorophenyl)quinolin-4-yl)((R)-piperidin-2-yl)methanol.

In one embodiment of this aspect, said mixture comprises at least 95% of (S)-(2-(4-chlorophenyl)quinolin-4-yl)((R)-piperidin-2-yl)methanol.

In one embodiment of this aspect, wherein said mixture comprises at least 99% of (S)-(2-(4-chlorophenyl)quinolin-4-yl)((R)-piperidin-2-yl)methanol.

In one embodiment of this aspect, there is provided said composition for use, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

In one embodiment of this aspect, there is provided said composition for use, wherein said systemic cancer is pancreatic cancer.

In one embodiment of this aspect, there is provided said composition for use, wherein said systemic cancer is skin cancer.

In one embodiment of this aspect, there is provided said composition for use, wherein side effects on central nervous system are minimized.

In another aspect of the invention, there is provided (S)-(2-(4-chlorophenyl)quinolin-4-yl)((R)-piperidin-2-yl)methanol, or a pharmaceutically acceptable salt or solvate thereof.

In another aspect of the invention, there is provided a method of stereoselective synthesis method of (R)-(2-(4-chlorophenyl)quinolin-4-yl)((S)-piperidin-2-yl)methanol, as set out in example 1.

In another aspect of the invention, there is provided a method of stereoselective synthesis method of (S)-(2-(4-chlorophenyl)quinolin-4-yl)((R)-piperidin-2-yl)methanol, as set out in example 2.

The compounds, salts and prodrugs of the present invention can exist in several tautomeric forms, and such tautomeric forms are included within the scope of the present invention. Tautomers exist as mixtures of a tautomeric set in solution. In solid form, usually one tautomer predominates. Even though one tautomer may be described, the present invention includes all tautomers of the present compounds.

As used herein, the term "salt" such as a pharmaceutically acceptable salt and can include acid addition salts including hydrochlorides, hydrobromides, phosphates, sulphates, hydrogen sulphates, alkylsulphonates, arylsulphonates, acetates, benzoates, citrates, maleates, fumarates, succinates, lactates, and tartrates; alkali metal cations such as Na⁺, K⁺, Li⁺, alkali earth metal salts such as Mg²⁺ or Ca²⁺, or organic amine salts.

By "pharmaceutically acceptable salt" it is meant those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describe pharmaceutically acceptable salts in detail (Berge, S. M. et al. J. Pharmaceutical Sciences, 1977, 66:1-19). The salts can be prepared in situ during the final isolation and purification of the compounds of the invention, or separately by reacting the free base function with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

Pharmaceutically acceptable salts include acid addition salts formed with inorganic acids, e.g. hydrochloric acid, hydrobromic acid, sulfuric acid, nitric acid, phosphoric acid; or formed with organic acids, e.g. acetic acid, benzenesulfonic acid, benzoic acid, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid; or salts formed when an acidic proton present in the parent compound either is replaced by a metal ion, e.g., an alkali metal ion, an alkaline earth ion, or an aluminum ion; or coordinates with an organic or inorganic base. Acceptable organic bases include e.g. diethanolamine, ethanolamine, N-methylglucamine, triethanolamine, and tromethamine. Acceptable inorganic bases include e.g. aluminum hydroxide, calcium hydroxide, potassium hydroxide, sodium carbonate and sodium hydroxide.

For the purpose of the present invention "pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

The term "effective amount" refers to an amount of a compound that confers a therapeutic effect on the treated patient. The effect may be objective (i.e. measurable by some test or marker) or subjective (i.e. the subject gives an indication of or feels an effect).

Pharmaceutically acceptable excipients for use in formulating a compound according to the invention as described and claimed herein, are for example, vehicles, adjuvants, carriers or diluents, which are well-known to those skilled in the art. Pharmaceutical excipients useful in formulating a compound as herein claimed and disclosed are found in e.g. Remington: The Science and Practice of Pharmacy, 19th ed., Mack Printing Company, Easton, Pennsylvania (1995).

The compounds of the invention can be administered by any suitable means, for example, orally, such as in the form of tablets, pills, dragees, aqueous or oily suspensions or solutions, elixirs, syrups, capsules, granules or powders; sublingually; buccally; parenterally, such as by e.g. subcutaneous, intravenous, intramuscular, or intrasternal injection or infusion techniques (e.g., as sterile injectable aqueous or non-aqueous solutions or suspensions). For parenteral administration, a parenterally acceptable aqueous or oily suspension, emulsion or solution is employed, which is pyrogen free and has requisite pH, isotonicity, osmolality and stability. Those skilled in the art are well able to prepare suitable formulations and numerous methods are described in the literature. A brief review of methods of drug delivery is also found in the scientific literature [eg. Langer, Science 249:1527-1533 (1990)].

Other examples of possible methods of administering the compounds of the invention are nasal administration including administration to the nasal membranes, such as by inhalation spray; or rectally such as in the form of suppositories; in dosage unit formulations containing non-toxic, pharmaceutically acceptable vehicles or diluents.

Preferably, the compounds of the present invention are parenterally administered in a way optimized for delivery to the brain of the treated subject. In one embodiment, the compounds are formulated for intraperitoneal administration. In one preferred embodiment, the compounds are formulated for intracerebroventricular administration.

The present compounds can also be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The compounds of the invention can also be administered liposomally. The precise nature of the carrier or other material will depend on the route of administration and those skilled in the art are well able to prepare suitable solutions and numerous methods are described in the literature. Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of the invention can also be delivered through the oral cavity by sublingual and/or buccal administration. Molded tablets, compressed tablets or freeze-dried tablets are exemplary forms, which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Exemplary compositions for nasal aerosol or inhalation administration include solutions in saline, which can contain, for example, benzyl alcohol or other suitable preservatives, absorption promoters to enhance bioavailability, and/or other solubilizing or dispersing agents such as those known in the art.

Exemplary compositions for parenteral administration include injectable solutions, emulsions or suspensions which can contain, for example, suitable non-toxic, parenterally acceptable diluents or solvents, such as mannitol, 1,3-butanediol, water, Ringer's solution, an isotonic sodium chloride solution, oil or other suitable dispersing or wetting and suspending agents, including synthetic mono- or diglycerides, and fatty acids, including oleic acid, or Cremaphor.

Exemplary compositions for rectal administration include suppositories, which can contain, for example, a suitable non-irritating excipient, such as cocoa butter, synthetic glyceride esters or polyethylene glycols, which are solid at ordinary temperatures, but liquify and/or dissolve in the rectal cavity to release the drug.

The dose administered to a mammal, particularly a human, in the context of the present invention should be sufficient to effect a therapeutic response in the mammal over a reasonable time frame. One skilled in the art will recognize that dosage will depend upon a variety of factors including the potency of the specific compound, the age, condition and body weight of the patient, the extent of the condition being treated, recommendations of the treating physician, and the therapeutics or combination of therapeutics selected for administration, as well as the stage and severity of the disease. The dose will also be determined by the route (administration form), timing and frequency of administration. Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 20 mg/kg/day, and most preferably 0.1 to 10 mg/kg/day, for adult humans. For oral administration, the compositions are preferably provided in the form of tablets or other forms of presentation provided in discrete units containing 0.5 to 1000 milligrams of the active ingredient for the symptomatic adjustment of the dosage to the patient to be treated, for example 0.5, 1.0, 2.5, 5.0, 10.0, 15.0, 25.0, 50.0, 100, 200, 400, 500, 600 and 800 mg.

Parenterally, especially intracerebroventricularly or intraperitoneally, the most preferred doses will range from about 0.001 to about 10 mg/kg/hour during a constant rate infusion. Advantageously, compounds of the present invention may be administered in single doses, e.g. once daily or more seldom, or in a total daily dosage administered in divided doses of two, three or four times daily.

Compounds of the present invention may also be used or administered in combination with at least one second therapeutic agent useful in the treatment of systemic cancer. The therapeutic agents may be in the same formulation or in separate formulations for administration simultaneously or sequentially. Compounds of the present invention may also be used in a combinational therapy or administered in combination with additional therapies, such as surgery and/or irradiation and/or other therapeutic strategies, including chemotherapies and immunotherapies.

The term "treatment" as used throughout the specification and claims encompasses preventive therapy, palliative therapy or curative therapy. Thus, the term "treating" (or treatment) encompasses not only treating (or treatment of) a patient to relieve the patient of the signs and symptoms of the disease or condition, or to ameliorate the condition of the patient suffering from the disease or disorder, but also prophylactically treating an asymptomatic patient to prevent the onset or progression of the disease or condition. In one embodiment, the treatment is to relieve the patient of the signs and symptoms of the disease or condition, or to ameliorate the condition of the patient suffering from the disease or disorder or to prevent progression of the disease or condition.

As used herein, "treating," "treatment" or "treat" describes the management and care of a patient for the purpose of combating a disease, condition, or disorder and includes the administration of a compound of the present invention, to alleviate the symptoms or complications of a disease, condition or disorder, or to eliminate the disease, condition or disorder. The term "treat" can also include treatment of a cell *in vitro* or an animal model.

The term "patient(s)" include mammalian (including human) patient(s) (or "subject(s)"). As used herein, a "subject" is interchangeable with a "subject in need thereof", both of which refer to a subject having a disorder in which viral infection plays a part, or a subject having an increased risk of developing cancer relative to the population at large. A "subject" includes a mammal. The mammal can be *e.g.,* a human or appropriate non-human mammal, such as primate, mouse, rat, dog, cat, cow, horse, goat, camel, sheep or a pig. In one embodiment, the mammal is a human.

As used in the present disclosure, whether in a transitional phrase or in the body of a claim, the terms "comprise(s)" and "comprising" are to be interpreted as having an open-ended meaning. That is, the terms are to be interpreted synonymously with the phrases "having at least" or "including at least." When used in the context of a process the term "comprising" means that the process includes at least the recited steps, but may include additional steps. When used in the context of a molecule, compound, or composition, the term "comprising" means that the compound or composition includes at least the recited features or components, but may also include additional features or components.

All percentages and ratios used herein, unless otherwise indicated, are by weight. Other features and advantages of the present invention are apparent from the different examples. The provided examples illustrate different components and methodology useful in practicing the present invention. The examples do not limit the claimed invention. Based on the present disclosure the skilled artisan can identify and employ other components and methodology useful for practicing the present invention.

### List of abbreviations

- 2-MeTHF: 2-Methyltetrahydrofuran
- API-ES: Atmospheric Pressue Ionization-Electrospray
- ATP: Adenosine Triphosphate
- AUC: Area Under Curve
- CNS: Central Nervous System
- DMEM: Dulbecco's Modified Eagle's Medium
- DMSO: Dimethysulfoxide
- EDTA: Ethylene Diamine Tetraacetic Acid
- ESI: Electrospray Ionization
- EtOAc: Ethyl Acetate
- Et₂O: Diethyl Ether
- Et₃N: Triethylamine
- FCS: Fetal Calf Serum
- HPLC-MS: High Performance Liquid Chromatography
- i.v.: Intravenous
- MS: Mass Spectrometry
- Na-CMC: Sodium Carboxymethyl Cellulose
- NCA: Non-Compartmental Analysis
- NCI: National Cancer Institute
- NMP: N-Methylpyrrolidine
- NMR: Nuclear Magnetic Resonance
- p.o.: per oral
- PBS: Phosphate Buffered Saline
- RAS: Rat sarcoma viral oncogene homolog
- RPMI: Roswell Park Memorial Institute medium
- TEA: Triethylamine
- TFA: Trifluoroacetic Acid
- THF: Tetrahydrofuran
- Tr: Triphenylmethyl

### Examples

The invention is hereby illustrated by the following non-limiting examples.

### Example 1.

### Stereoselective synthesis of (R)-(2-(4-chlorophenyl)quinolin-4-yl)((S)-piperidin-2-yl)methanol (S1)

A stereoselective synthesis of **S1** was designed based on a modification of León (León, B., et al (2013). Organic Letters, 15(6), 1234-7), according to following Scheme 2.

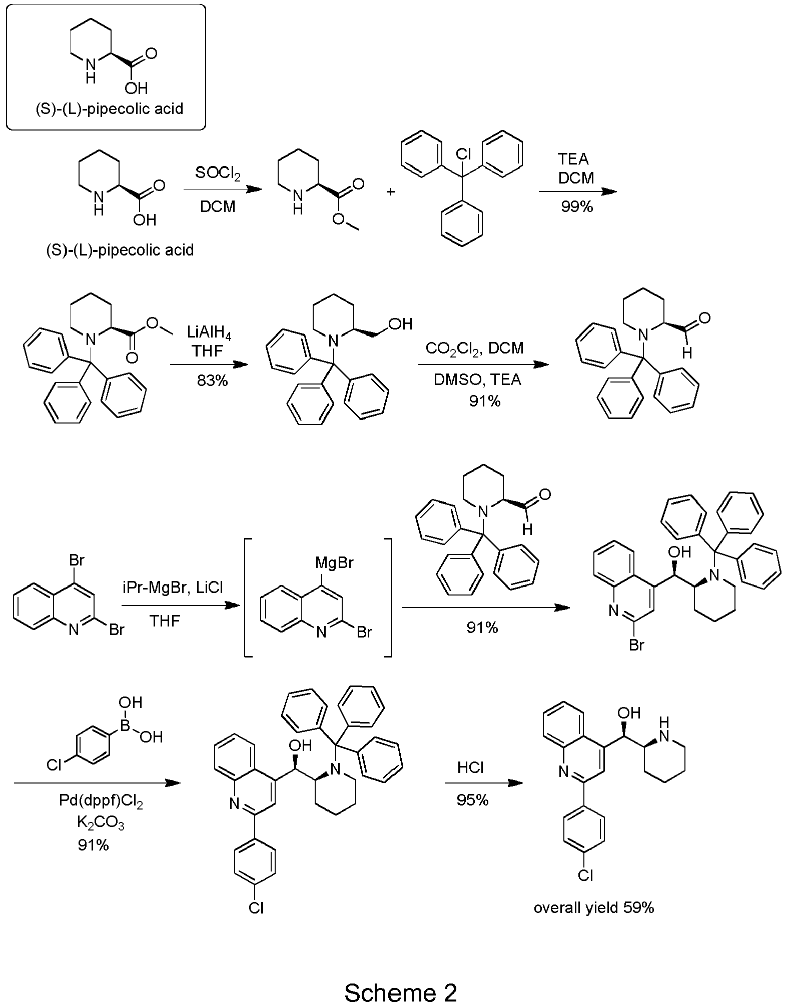

Briefly, tritylation of methylated (S)-L-Pipecolic acid afforded the possibility to generate a chiral piperidine carbaldehyde material suitable for face-selective addition by the Grignard reagent generated from 2,4-dibromoquinoline. The single isolated R,S isomer was then subject to Suzuki coupling of the appropriate 4-chlorophenylboronic acid, which after concomitant deprotection of the trityl group yields the desired (R)-[2-(4-chlorophenyl)quinolin-4-yl](2S)-piperidin-2-ylmethanol **(S1).**

### Methyl (2S)-piperidine-2-carboxylate

(S)-(L)-Pipecolic acid (6.1 g, 47.2 mmol) was added to methanol (47.2 mL) under an N₂ atmosphere. To this solution thionyl chloride (6.9 mL, 94.3 mmol) was slowly added at -10 °C. The reaction mixture was allowed to warm to rt and was stirred for 18 hours. Reaction mixture was evaporated and co-evaporated with toluene and dried under vacuum. The crude methyl (2S)-piperidine-2-carboxylate was used in next step without further purification or characterization.

### Methyl (2S)-1-(triphenylmethyl)piperidine-2-carboxylate

Methyl (2S)-piperidine-2-carboxylate (6.8 g, 47.2 mmol) was dissolved in CH₂Cl₂ (57 mL), then Et₃N (19.7 mL, 141.5 mmol) was added. To this solution was added trityl chloride (13.1 g, 47.2 mmol) and the reaction mixture was stirred for 18 h at rt. The reaction was quenched with NH₄Cl/28% NH₃ (40 mL, 2:1). The solution was partitioned between Et₂O (80 mL) and H₂O (80 mL). The layers were separated and the aqueous layer was extracted with Et₂O (3 x 50 mL). The combined organic layers were dried with Na₂SO₄, filtered, and concentrated *in vacuo.* The residue was purified by flash chromatography (1:2:97, Et₃N:EtOAc:Heptane) to give title compound (18.2 g, 47.2 mmol, 99% yield) as a white foam. MS (ESI+) *m*/*z* 144 [M+H-Tr]+.

### [(2S)-1-(triphenyimethyl)piperidin-2-yl]methanol

To an oven dried 3-neck flask equipped with a stir bar and condenser was added THF (55 mL) under an N₂ atmosphere. To this solution was added LiAlH₄ (3.2 g, 86.2 mmol) and the reaction was allowed to stir to form a suspension. To this suspension was added methyl (2S)-1-(triphenylmethyl)piperidine-2-carboxylate (22.1 g, 57.4 mmol). The reaction solution was allowed to stir for 3h at rt (became thick suspension after 30 min and 10 ml THF was added). The reaction mixture was then cautiously quenched with NaOH (11 mL, 1.0 M), and H₂O (22 mL). The solution became visibly thicker and more difficult to stir. MgSO₄ was then added and the solution was passed through a pad of celite with 300 mL of dichloromethane. This was then concentrated *in vacuo.* The residue was purified by flash chromatography (1:1:98, Et₃N:MeOH: CH₂Cl₂) to yield the title compound (17 g, 47.6 mmol, 83% yield) as a white foam. MS (ESI+) *m*/*z* 116 [M+H-Tr]^{+ 1}H NMR (400 MHz, DMSO-d₆) δ 7.42 (d, *J* = 7.58 Hz, 6H), 7.28 (t, *J* = 7.74 Hz, 6H), 7.15 (t, *J* = 7.70 Hz, 3H), 3.93 - 3.99 (m, 1 H), 3.11 - 3.24 (m, 2H), 2.74 - 2.84 (m, 1 H), 2.26 - 2.38 (m, 2H), 1.60 - 1.76 (m, 2H), 1.43 - 1.59 (m, 2H), 1.35 (br. s., 2H). ¹³C NMR (101 MHz, DMSO-d₆) δ 144.9, 128.8, 127.7, 125.8, 77.2, 59.7, 56.1, 54.5, 42.1, 25.1, 18.6.

### (2S)-1-(triphenylmethyl)piperidine-2-carbaldehyde

To an oven dried flask equipped with a stir bar under an N₂ atmosphere was added CH₂Cl₂ (57 mL) and the reaction was cooled to -78 °C. To this solution was slowly added oxalyl chloride (6.1 mL, 71.3 mmol). Next a solution of DMSO (8.4 mL, 118.9 mmol) in CH₂Cl₂ (33 mL) was added dropwise. This was allowed to stir for 10 min and a solution of [(2S)-1-(triphenylmethyl)piperidin-2-yl]methanol (17 g, 47.6 mmol) in CH₂Cl₂ (43 mL) was added. The suspension was allowed to stir for 2 h and then Et₃N (26.5 mL, 190.2 mmol) was added and allowed to stir for an additional 3 h. The -78 °C bath was then removed and NH₄Cl/28% NH₃ (100 mL, 2:1) was added and the solution was partitioned between CH₂Cl₂ (60 mL) and H₂O (60 mL). The layers were separated and the aqueous layer was extracted with CH₂Cl₂ (3 x 70 mL). The combined organic layers were dried with Na₂SO₄, filtered, and concentrated *in vacuo.* The residue was purified by flash chromatography (1:9:90, Et3N:EtOAc:Heptane) to afford title compound (1 g, 91 %) as a white solid. MS (ESI+) *m*/*z* 114 [M+H-Tr]⁺. ¹H NMR (400 MHz, DMSO-d₆) δ 8.43 (s, 1 H), 7.42 (d, *J* = 7.42 Hz, 6H), 7.32 (t, *J* = 7.74 Hz, 6H), 7.16 - 7.24 (m, 3H), 3.55 (d, *J* = 1.74 Hz, 1 H), 3.12 - 3.21 (m, 1 H), 2.77 (s, 1 H), 1.86 - 2.00 (m, 1 H), 1.74 - 1.83 (m, 1 H), 1.62 - 1.74 (m, 1 H), 1.57 (d, *J =* 11.22 Hz, 2H), 1.01 - 1.18 (m, 1 H). ¹³C NMR (101 MHz, DMSO-d₆) δ 208.9, 143.4, 128.6, 128.2, 126.3, 76.9, 62.0, 43.1, 28.9, 25.2, 20.7.

### (R)-(2-bromoquinolin-4-yi)[(2S)-l-(triphenylmethyl)piperidin-2-yl]methanol

2,4-dibromoquinoline (5.0 g, 17.6 mmol) was dissolved in dry tetrahydrofuran (42 mL). i-PrMgCl LiCl complex 1.3 M solution in tetrahydrofuran (16.0 mL, 20.8 mmol) was added slowly, drop-wise, at -5 - 0°C. The reaction mixture was stirred at rt for 20 min. (2S)-1-(triphenylmethyl)piperidine-2-carbaldehyde (5.7 g, 16.0 mmol, 1.0 equiv.) was added in one portion at 0°C and reaction was allowed to reach room temperature and the reaction mixture was stirred at rt for 5h. After the reaction was completed NH₄Cl (sat.)/NH₃(28%) (30 ml) solution was added and the mixture was extracted with CH₂Cl₂ (3 x 20 mL). The organic phase was separated and washed with brine (20 mL). The solution was dried over Na₂SO₄, filtered and then evaporated in vacuo. The resultant was chromatographed on silica gel eluting with TEA:ethyl acetate:heptane (1:10:90). Crude was dissolved in TEA/ethyl acetate/heptane (1:10:90) and EtOAc was added to get solution. SiO₂ was added to the solution and the mixture was evaporated to dryness and was added to SiO₂ column (d=7 cm x h=10 cm) eluting with TEA/ethyl acetate/heptane (1:10:90) to (1:10:40). Fractions were collected and dried under vacuum to give title compound (8.2 g, 14.5 mmol, 91% yield) as a white solid. Exact mass for C₃₄H₃₂BrN₂O [M+H]⁺ requires *m*/*z* 563.1698, HPLC-MS (API-ES) (ACE C8 10-90% MeCN 1.5 min (0.1 % TFA pH 2) (API-ES) C₁₅H₁₈ClN₂O [M+H]+ requires m/z 321.0602 found m/z 321, (Trityl-group was removed in acidic condition). ). ¹H NMR (400 MHz, DMSO-d₆) δ 7.92 - 7.98 (m, 1 H), 7.81 (s, 1 H), 7.70 - 7.76 (m, 1 H), 7.38 (d, *J =* 6.95 Hz, 6H), 7.24 (s, 1 H), 7.12 - 7.22 (m, 9H), 6.01 (d, *J* = 4.74 Hz, 1 H), 5.72 - 5.80 (m, 1 H), 3.68 - 3.76 (m, 1 H), 3.60 - 3.68 (m, 1 H), 2.95 - 3.05 (m, 1 H), 1.65 - 1.83 (m, 1 H), 1.20 - 1.31 (m, 1 H), 0.95 - 1.13 (m, 4H), 0.07 - 0.28 (m, 1H). ¹³C NMR (101 MHz, DMSO-d₆) δ 154.9, 148.1, 144.8, 141.6, 130.3, 129.8, 128.7, 127.6, 126.6, 126.0, 124.8, 124.7, 123.8, 78.4, 73.6, 55.6, 31.1, 22.6, 18.5, 14.0.

### (R)-[2-(4-chlorophenyl)quinolin-4-yl][(2S)-1-(triphenylmethyl)piperidin-2-yl]methanol

(R)-(2-bromoquinolin-4-yl)[(2S)-1-(triphenylmethyl)piperidin-2-yl]methanol (7.9 g, 14.0 mmol) and 4-chlorophenylboronic acid (2.3 g, 14.7 mmol) were dissolved in 2-MeTHF (5.5 mL) under N₂, PdCl₂(dppf) (0.23 g, 0.28 mmol) and 2M K₂CO₃ (28.0 mL, 55.8 mmol) were added under nitrogen atmosphere and the reaction was heated at 80 °C for 5h, conversion followed by HPLC. Phases was separated and organic phase was evaporated to dryness and dissolved in EtOAc (7 mL) and heptane (10 mL) was added. The resulting solution was filtered through silica gel eluting with triethylamine/ethyl acetate/heptane (1:20:80). First 400 mL containing product was evaporated to give the title compound (7.6 g, 12.7 mmol, 91 % yield). HPLC-MS (API-ES) Exact mass for C₄₀H₃₅ClN₂O [M+H]⁺ requires *m*/*z* 594.2437. HPLC-MS (API-ES) (ACE C8 10-90% MeCN 1.5 min (0.1% TFA pH 2) (API-ES) C₂₁H₂₁ClN₂O [M+H]⁺ requires *m*/*z* 353.1421, found *mlz* 353, (Trityl-group was removed in acidic condition). ¹H NMR (400 MHz, DMSO-d₆) δ 8.29 - 8.36 (m, 3H), 8.08 (dd, *J* = 0.95, 8.53 Hz, 1 H), 7.71 (ddd, *J* = 1.11, 6.95, 8.37 Hz, 1 H), 7.64 - 7.68 (m, 2H), 7.46 (br. d, *J* = 8.50 Hz, 1 H), 7.30 - 7.38 (m, 6H), 7.21 (ddd, *J* = 1.26, 6.95, 8.37 Hz, 1 H), 7.10 (d, *J* = 2.21 Hz, 8H), 5.94 (d, *J* = 4.74 Hz, 1 H), 5.85 (t, *J* = 5.29 Hz, 1 H), 3.71 - 3.82 (m, 2H), 2.95 - 3.05 (m, 1 H), 1.73 - 1.88 (m, 1 H), 1.20 - 1.30 (m, 2H), 1.08 - 1.15 (m, 1 H), 0.97 - 1.07 (m, 2H), 0.08 - 0.25 (m, 1 H).

### (R)-[2-(4-chlorophenyl)quinolin-4-yl](2S)-piperidin-2-ylmethanol dihydrochloride (S1)

(R)-[2-(4-chlorophenyl)quinolin-4-yl][(2S)-1-(triphenylmethyl)piperidin-2-yl]methanol (7.6 g, 12.7 mmol) was dissolved in Et₂O (114 mL) followed by addition of 3M HCl (197 mL). After stirring at room temperature for 4 h, the solution was partitioned between Et₂O (70 mL) and H₂O (400 mL). The aqueous layer was extracted with Et₂O (3 x 80 mL). The aqueous layer was then basified with 6M NaOH (50 mL gave pH 12-13), and then was extracted with CH₂Cl₂ (200 mL). The CH₂Cl₂ layer was dried with Na₂SO₄, filtered, and concentrated in vacuo. Product was solved in CH₂Cl₂ 50 ml and 1 M HCl in Et₂O (12 ml) was added droppwise at 0 °C under stirring, precipitate was collected and dried under vacuum to give title compound dihydrochloride salt (5.1 g, 12.0 mmol, 95% yield) as a white solid. HPLC-MS (API-ES) Exact mass for C₂₁H₂₁ClN₂O [M+H]⁺ requires *m*/*z* 353.1421, found m/z353. ¹H NMR (400 MHz, DMSO-d₆) δ 10.45 (d, *J* = 9.79 Hz, 1 H), 8.60 (d, *J* = 8.37 Hz, 1 H), 8.47 (q, *J* = 10.37 Hz, 1 H), 8.25 - 8.31 (m, 3H), 8.22 (s, 1 H), 7.87 - 7.94 (m, 1 H), 7.73 (ddd, *J* = 1.11, 7.03, 8.29 Hz, 1 H), 7.66 - 7.70 (m, 2H), 6.07 (d, *J* = 1.90 Hz, 1 H), 3.35 - 3.46 (m, 1 H), 3.24 (d, *J* = 11.69 Hz, 1 H), 2.89 - 3.03 (m, 1 H), 1.50 - 1.78 (m, 3H), 1.16 - 1.34 (m, 1 H).

### Example 2.

### Stereoselective synthesis of (S)-(2-(4-chlorophenyl)quinolin-4-yl)((R)-piperidin-2-yl)methanol (S2)

A stereoselective synthesis of S2 was performed according to the synthesis of S1 but starting with the corresponding (R)-(D)-pipecolic acid according to Scheme 3.

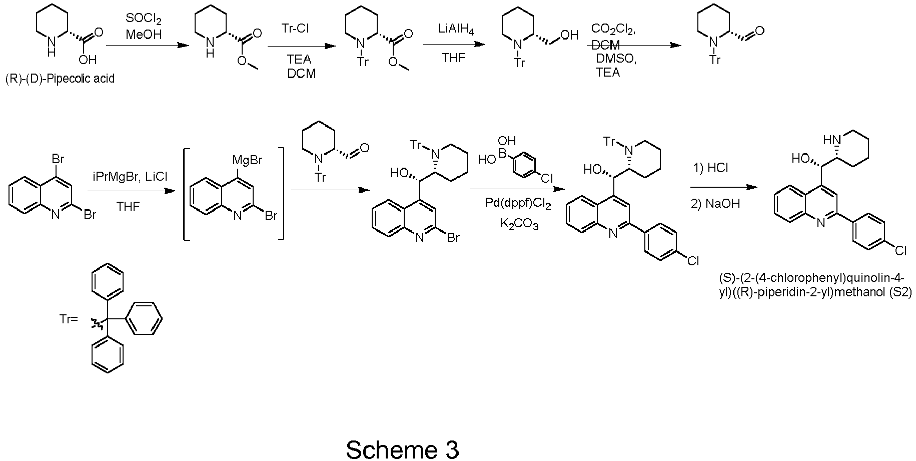

Briefly, tritylation of methylated (R)-(L)-pipecolic acid afforded the possibility to generate a protected chiral piperidine carbaldehyde material suitable for face-selective addition by the Grignard reagent generated from 2,4-dibromoquinoline. The isolated *R,S* isomer addition product was then subject to Suzuki coupling with 4-chlorophenylboronic acid, which after concomitant deprotection of the trityl group yields the desired (S)-(2-(4-chlorophenyl)quinolin-4-yl)((*R*)-piperidin-2-yl)methanol **(S2).**

### (R)-Methyl piperidine-2-carboxylate

(*R*)-(D)-Pipecolic acid (15 g, 116 mmol) was dissolved in methanol (120 mL) under an N₂ atmosphere. To this solution thionyl chloride (17 mL, 232 mmol) was slowly added at -10 °C. The reaction mixture was allowed to warm to room temperature and stirred for 18 hours. The reaction mixture was concentrated and co-evaporated with toluene. The crude (R)-methyl piperidine-2-carboxylate was dried under vacuum and was then used in the next step without further characterization or purification.

### (R)-Methyl 1-tritylpiperidine-2-carboxylate

(R)-Methyl piperidine-2-carboxylate (17 g, 116 mmol) was dissolved in CH₂Cl₂ (140 mL) and triethylamine (49 mL, 348 mmol) was added. To this mixture was added trityl chloride (TrCl) (32 g, 116 mmol) and the reaction mixture was stirred for 18 hrs at room temperature. The reaction was quenched with NH₄Cl/28% NH₃ (100 mL, 2:1) and partitioned between Et₂O (80 mL) and H₂O (80 mL). The layers were separated and the aqueous layer was extracted with Et₂O (2 x 50 mL). The combined organic layers were dried over Na₂SO₄, filtered, and concentrated in vacuo. The residue was purified by flash chromatography (1:2:97, Et₃N:EtOAc:Heptane) to give the title compound (37 g, 96 mmol, 83% yield) as a white foam. MS (ESI⁺) *m*/*z* 144 [M-Tr+H]⁺. ¹H NMR (400 MHz, DMSO-d₆) δ 7.36 (d, *J* = 1.00 Hz, 6H), 7.27 (t, *J* = 7.82 Hz, 7H), 7.15 (t, *J* = 7.30 Hz, 3H), 3.93 - 3.99 (m, 1 H), 3.14 - 3.22 (m, 1 H), 3.13 (s, 3H), 2.86 - 2.93 (m, 1 H), 2.00 - 2.12 (m, 1 H), 1.62 - 1.71 (m, 1 H), 1.51 - 1.62 (m, 3H), 1.22 - 1.40 (m, 1 H).

### (R)-(1-Tritylpiperidin-2-yl)methanol

To an oven dried 3-neck flask equipped with a condenser and stirring bar was added THF (100 mL), LiAlH₄ (5 g, 144 mmol) under N₂. To this suspension was added drop-wise (R)-methyl 1-tritylpiperidine-2-carboxylate (37 g, 96 mmol) in 120 mL THF, (exothermic, gave 60 °C). The reaction solution was allowed to stir for 3 hrs at room temperature. (Thick suspension was obtained after 30 min and 50 ml THF was added). The reaction mixture was carefully quenched with drop-wise addition of NaOH (19 mL, 1 M) and H₂O (37 mL). MgSO₄ was added and the solution was filtered through a pad of Celite, which was washed with 100 mL of ethyl acetate. The combined organic fractions were concentrated in vacuo, to give the title compound (33 g, 94 mmol, 98% yield) as a white foam. MS (ESI⁺) *m*/*z* 116 [M-Tr+H]⁺. ¹H NMR (400 MHz, DMSO-d₆) δ 7.43 (d, *J* = 7.42 Hz, 6H), 7.28 (t, *J* = 7.74 Hz, 6H), 7.13 - 7.18 (m, 3H), 3.96 (t, *J =* 6.00 Hz, 1 H), 3.18 - 3.25 (m, 1 H), 3.17 (d, *J =* 3.79 Hz, 1 H), 2.78 (br. s., 1 H), 2.32 (br. s., 1 H), 2.30 (s, 1 H), 1.70 (br. s., 2H), 1.48 (br. s., 2H), 1.36 (d, *J* = 4.74 Hz, 2H).

### (R)-1-Tritylpiperidine-2-carbaldehyde

Oxalyl chloride (11 mL, 131 mmol) was added to dichloromethane (105 mL) in an oven dried flask equipped with a stir bar under (N₂) at -78 °C. A solution of DMSO (16 mL, 219 mmol) in dichloromethane (61 mL) was added drop-wise. The mixture was allowed to stir for 10 min and a solution of (*R*)-(1-tritylpiperidin-2-yl)methanol (31 g, 88 mmol) in dichloromethane (80 mL) was added. The suspension was allowed to stir for 2 hrs and then triethylamine (49 mL, 350 mmol) was added and allowed to stir for an additional 3 hrs. The -78 °C bath was then removed and NH₄Cl/28% NH₃ (100 mL, 2:1) was added and the solution was partitioned between dichloromethane (60 mL) and H₂O (60 mL). The layers were separated and the aqueous layer was extracted with dichloromethane (2 x 70 mL). The combined organic layers were dried with Na₂SO₄, filtered, and concentrated in vacuo. The residue was purified by flash chromatography (1:9:90, Et₃N:EtOAc:Heptane) to afford the title compound (22 g, 62 mmol, 71%) as a white solid. MS (ESI⁺)*m*/*z* 114 [M+H-Tr]⁺. ¹H NMR (400 MHz, DMSO-d₆) δ 7.42 (d, *J* = 7.58 Hz, 6H), 7.32 (t, *J =* 7.90 Hz, 6H), 7.16 - 7.23 (m, 3H), 3.52 - 3.59 (m, 1H), 3.11 - 3.21 (m, 1 H), 2.73 - 2.83 (m, 1 H), 1.86 - 2.00 (m, 1 H), 1.74 - 1.83 (m, 1 H), 1.62 - 1.74 (m, 1 H), 1.50 - 1.62 (m, 2H), 1.02 - 1.18 (m, 1 H).

### (S)-(2-bromoquinolin-4-yl)((R)-1-tritylpiperidin-2-yl)methanol

2,4-Dibromoquinoline (6.9 g, 24 mmol) was dissolved in dry THF (65 mL). i-PrMgCI LiCl complex 1.3 M solution in THF (22 mL, 28 mmol) was added slowly, drop-wise, at -5 - 0 °C. The reaction mixture was stirred at 0 °C for 20 min. (*R*)-1-Tritylpiperidine-2-carbaldehyde (7.8 g, 22 mmol) was added in one portion at 0 °C and the reaction mixture was allowed to reach room temperature and the reaction mixture was stirred at room temperature for 5 hrs. NH₄Cl (sat.)/NH₃(28%) (100 mL) solution was added and the mixture was extracted with ethyl acetate (1 x 50 mL). The organic phase was separated and washed with brine (20 mL). The solution was dried over Na₂SO₄, filtered and then concentrated in vacuo. The residue was purified by column chromatography (SiO₂) eluting with TEA:ethyl acetate:heptane (1:10:90). Fractions were collected, concentrated and dried under vacuum to give title compound (11.4 g, 20 mmol, 93% yield) as a white solid. MS (ESI⁺) *m*/*z321,* [M-Tr+H]⁺. ¹H NMR (400 MHz, DMSO-d₆) δ 7.95 (d, *J =* 8.37 Hz, 1 H), 7.81 (s, 1 H), 7.69 - 7.76 (m, 1 H), 7.39 (d, *J* = 6.95 Hz, 6H), 7.24 (s, 2H), 7.10 - 7.21 (m, 9H), 6.00 (d, *J* = 4.74 Hz, 1 H), 5.76 (t, *J* = 5.05 Hz, 1 H), 3.59 - 3.76 (m, 2H), 2.92 - 3.05 (m, 1 H), 1.67 - 1.83 (m, 1 H), 0.96 - 1.13 (m, 4H), 0.10 - 0.29 (m, 1 H)

### (S)-(2-(4-Chlorophenyl)quinolin-4-yl)((R)-1-tritylpiperidin-2-yl)methanol

(S)-(2-Bromoquinolin-4-yl)((*R*)-1-tritylpiperidin-2-yl)methanol (8.5 g, 15 mmol) and 4-chlorophenylboronic acid (2.5 g, 16 mmol) were dissolved in 2-MeTHF (105 mL) under an N₂ atmosphere. PdCl₂(dppf) (0.25 g, 0.3 mmol) and 2M K₂CO₃ (30 mL, 60 mmol) were added and the reaction was heated at 70 °C for 7 hrs. The organic phase was separated and evaporated in vacuo. The residue was dissolved in EtOAc (7 mL) and heptane (10 mL) was added. The resulting solution was filtered through silica gel eluting with triethylamine/ethyl acetate/heptane (1:10:90). The first 400 mL containing product was evaporated to give the title compound (9.0 g, 15 mmol, 99% yield). MS (ESI⁺ *m*/*z* 353, [M-Tr+H]⁺. ¹H NMR (400 MHz, DMSO-d₆) δ 8.27 - 8.35 (m, 3H), 8.08 (dd, *J* = 1.03, 8.45 Hz, 1 H), 7.59 - 7.74 (m, 3H), 7.03 - 7.47 (m, 18H), 5.94 (d, *J* = 4.58 Hz, 1 H), 5.82 - 5.90 (m, 1 H), 3.69 - 3.86 (m, 2H), 2.92 - 3.09 (m, 1 H), 1.72 - 1.92 (m, 1 H), 1.23 (d, *J* = 0.79 Hz, 2H), 0.96 - 1.15 (m, 3H), 0.07 - 0.27 (m, 1 H).

### (S)-(2-(4-chlorophenyl)quinolin-4-yl)((R)-piperidin-2-yl)methanol (S2)

(S)-[2-(4-chlorophenyl)quinolin-4-yl][(2R)-1-(trityl)piperidin-2-yl]methanol, (8.5 g, 14.3 mmol) was dissolved in diethyl ether (221 mL) followed by addition of 2M HCl (127 mL). After stirring at room temperature for 1 hour, the solution was partitioned between Et₂O (70 mL) and H₂O (400 mL). The aqueous layer was extracted with Et₂O (3 x 80 mL). The aqueous layer was then basified with 6M NaOH to give pH 12-13, then extracted with dichloromethane (2 x 70 mL). The organic fractions were combined and dried with Na₂SO₄, filtered, concentrated and dried under vacuum to give title compound **S2** (4.9 g, 14 mmol, 98% yield) as a white solid. MS (ESI⁺) *m*/*z* 353, [M+H]⁺. ¹H NMR (400 MHz, DMSO-d₆) δ 8.25 - 8.29 (m, 2H), 8.24 (d, *J* = 7.90 Hz, 1 H), 8.14 (s, 1 H), 8.09 (dd, *J* = 0.95, 8.53 Hz, 1 H), 7.77 (ddd, *J* = 1.26, 6.91, 8.41 Hz, 1 H), 7.58 - 7.65 (m, 3H), 5.65 (br. s., 1 H), 5.25 (d, *J =* 2.21 Hz, 1 H), 2.90 (d, *J* = 12.16 Hz, 1 H), 2.80 - 2.87 (m, 1 H), 2.37 - 2.47 (m, 1 H), 1.70 (d, *J* = 9.79 Hz, 1 H), 1.51 (d, *J* = 11.37 Hz, 1 H), 1.38 - 1.46 (m, 1 H), 1.08 - 1.31 (m, 3H).

### Example 3.

### Pharmacokinetic evaluation of S1 and S2

### Methods

Compounds **S1** and **S2** were formulated at 0.4 mg/mL in 7.5% NMP, 10% Kolliphor in 0.9% NaCl (for i.v. administration) and at 2.0 mg/mL in 0.1% Tween 80, 0.5% Na-CMC in water (for p.o. administration).

The pharmacokinetic properties of **S1** and **S2** were determined in male NMRI mice (Charles River, Germany) following single intravenous (i.v., tail vein, 2 mg/kg) or per oral (p.o, gavage, 20 mg/kg). Light isoflurane anaesthesia was used at i.v. administration, no anaesthesia was used at p.o. administration. Blood samples were kept on ice after sampling and centrigued at +4 oC, 1800 x g for 5 minutes to prepare plasma. Blood and homogenized brain samples were taken from animals at the following nominal time points: 15, 30, and 60 minutes, and 2, 4, 6, 8, 24, 48, 72 and 144 hours after dosing (n=3 samples/time-point). Blood sampling (400-600 microliters) was performed by orbital plexus collection under isoflurane anaesthesia into EDTA plasma tubes (BD Microtainer K2EDTA). Bioanalytical quantification of **S1** and **S2** was analysed in plasma and brain samples by a UPLC-MS/MS. Pharmacokinetics were calculated by non-compartmental analysis (NCA) from composite (mean) profiles using Phoenix WinNonlin Professional (v 6.3). Nominal sampling times and dose levels have been used for the NCA calculations. All animals dosed with **S1** and **S2** were systemically exposed to the test compound. The plasma and brain concentrations were detectable and analysed until 144 h.

Brain sampling was performed after the end of blood sampling at the specified time points following euthanization and skull extraction. Brains were weighed and placed in test tubes and placed on wet ice. 4 mL PBS (pH 7.4) per 1 g of brain tissue was added to the test tube and brains were then homogenized using an Ultra-Turrax T25 homogenisator (Setting 2, 9500 rpm, 10s). The homogenates were frozen at -20° C immediately after homogenization.

It was shown that the plasma/CNS pharmacokinetic exposure profiles of the most potent isomers, (R)-(2-(4-chlorophenyl)quinolin-4-yl)((S)-piperidin-2-yl)methanol **(S1)** and (S)-(2-(4-chlorophenyl)quinolin-4-yl)((R)-piperidin-2-yl)methanol **(S2),** differ substantially. While compound **S1** showed preferential exposure to brain tissue (exposure ratio C_{max(brain/plasma}): 2.2; exposure ratio AUC_{(brain/plasma)}: 1.6), compound **S2** has significantly lower exposure to CNS tissue (exposure ratio C_{max(brain/plasma)}: 0.7; exposure ratio AUC_{(brain/plasma)}: 0.9) after single oral administration of 20 mg/kg. Results are presented in Table 1.

**Table 1. In vivo pharmacokinetic parameters after administration of 2 (i.v.) or 20 (p.o.) mg/kg S1 and S2 to mice.**

| | | | **Tissue** | | | | | | | | | |
|---|---|---|---|---|---|---|---|---|---|---|---|---|
| | | | **Brain** | | | | | **Plasma** | | | | |
| Dose mg/kg | Isomer | Route | Cmax ng/mL | tₘₐₓ (h) | AUC last h*ng/mL | tₗₐₛₜ (h) | T_{1/2} (h) | Cmax ng/mL | tₘₐₓ (h) | AUC last h*ng/mL | tₗₐₛₜ (h) | T_{1/2} (h) |
| 2 | S1 | i.v. | 1970 | 0.25 | 52700 | 144 | 67 | 775 | 4.0 | 62000 | 144 | 84 |
| 2 | S2 | i.v. | 777 | 1.0 | 9050 | 72 | - | 455 | 0.5 | 14100 | 72 | - |
| 20 | S1 | p.o. | 4840 | 8.0 | 400000 | 144 | - | 2210 | 4.0 | 246000 | 144 | - |
| 20 | S2 | p.o. | 1490 | 6.0 | 157000 | 144 | - | 2050 | 8.0 | 183000 | 144 | - |

It was also shown that isomers **S1** and **S2** exhibit greater *in vitro* oncolytic potency than the corresponding **S3** and **S4** isomers on patient-derived U3013 glioma cells (see figure 7). Thus, it is concluded to be prefered to use **S1** and **S2** isomers for treatment of cancer.

Based on the identified anti-cancer activity of **S1** and **S2,** the use of **S2** for the treatment of systemic cancers such as pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, prostate, intestinal, skin, hematological/lymphoid, cervical, pancreatic, lung, thyroid, urinary tract, colorectal, salivary, prostate, intestinal, skin, hematological/lymphoid, pleura mesothelioma, renal, skin, sarcoma, soft tissue, testicular, uterine body cancer, is preferential as dose required for systemic exposure would minimize brain exposure and thus present lower risk of CNS-related side-effects.

### Example 4.

### In vitro testing of S1 and S2 using 3D and 2D assays

### Methods

### Cell cultures

The following well-characterized human pancreatic ductal adenocarcinoma cell lines (ATCC) were used: HPAF II, BxPC-3, MIA-PaCa2 and Panc-1. These cell lines were generated from patient-derived tumors and are widely used model system to study pancreatic cancer (HPAF II: Kim YW, et al. Pancreas 4: 353-362, 1989; BxPC-3: Tan MH, et al. Cancer Invest. 4: 15-23, 1986; MIA-PaCa2: Yunis AA, et al. Int. J. Cancer 19: 128-135, 1977; Pacn-1: Lieber M, et al. Int. J. Cancer 15: 741-747, 1975).

KPC cells were established from a mouse PDAC model, carrying pancreas-specific Kras and p53 mutations (KrasLSL-G12D/+; Trp53LSL-R172H/+;p48-Cre; hence KPC (see Torres et al., PLoS One. 2013 Nov 20;8(11):e80580. doi: 10.1371/journal.pone.0080580). Cells were cultured under standard culture conditions (5% CO2, at 37°C) in DMEM/F12 or phenol red-free DMEM/F12 medium (Gibco) containing 10% fetal calf serum (FCS, Invitrogen).

The human melanoma cell line SK-MEL-28 is a well established reference cell line in melanoma research (Goodfellow M, et al. J. Natl. Cancer Inst. 59: 221-226, 1977). Cells were cultured in MEM supplemented with 10% FCS. Glioblastoma cell line (U3013) was generated directly from patient-derived tumors (Kitambi et al. Cell. 2014: 157(2):313-28) and grown in serum-free media supplemented with N2, B27, EGF, and FGF-2 (20 ng/ml) using previously described methodology (Pollard et al., 2009).

Culture plates were pre-coated with laminin (Sigma) for 3 hr at 10 ug/ml prior to use and confluent cells were split 1:3 to 1:5 using TrypLE Express (Invitrogen).

### 3D culture

Panc-1, HPAF II, KPC pancreatic adenocarcinoma cell lines were trypsin-treated and counted using the Casy Cell Counter according to the manufacturer's recommendations (GmbH). Subsequently, they were seeded onto round bottom non-tissue culture treated 96 well-plates (Falcon, BD NJ, USA) at a concentration of 2500 cells/well in 100 µl DMEM-F12 or phenol red-free DMEM-F12 medium, containing 10% FCS and supplemented with 20% methyl cellulose stock solution. For preparation of methylcellulose stock solution, 6 grams of methylcellulose powder (M0512, Sigma-Aldrich) was autoclaved in a 500 ml flask containing a magnetic stirrer (the methylcellulose powder is resistant to this procedure). The autoclaved methylcellulose was dissolved in preheated 250 ml basal medium (60°C) for 20 min (using the magnetic stirrer). Thereafter, 250 ml medium (room temperature) containing double amount of FCS (20%) was added to a final volume of 500 ml and the whole solution mixed overnight at 4°C. The final stock solution was aliquoted and cleared by centrifugation (5000g, 2 h, room temperature). Only the clear highly viscous supernatant was used for the spheroid assay (about 90-95% of the stock solution). For spheroid generation, 20% of the stock solution and 80% culture medium corresponding to final 0.24% methylcellulose was used. Spheroids were grown under standard culture conditions (5% CO₂, at 37°C).

### Experimental design for S1 test in pancreatic adenocarcinoma cell lines

For 2D culture, cells were seeded on flat bottom 96 well plates (Costar) at a concentration of 2500 cells/well in 100 µl phenol red-free RPMI-F12 medium containing 10% FCS. For 3D culture, cells were seeded according to the description for spheroid preparation in phenol-free medium. On day 4 Vacquinol-1 RS **(S1)** was added at the indicated final concentrations in an extra volume of 10 µl/well and in 8 replicates for each time point. On day 7, an APH assay (See below) was performed. The viability rate was calculated as a percentage of the untreated cells. All data were expressed as the mean ± SD of at least 8 replicates. To confirm the reliability of the APH assay on 2D culture, cell viability was measured using CellTiter-Glo assay (Promega).

### Cell viability

Viability assay was conducted in 96 well microtiter plates seeded with 3000 cells. Cell viability was measured using CellTiter-Glo assay (Promega), according to the manufacturer's instructions. Luminescence was measured using Victor3 FA (Perkin Elmer) microtiter plate reader. Total luminescence was normalized relative to negative control and curve fitting performed using GraphPad Prism (v6.02) software.
IC50 was determined using Cell Viability assay, and data was illustrated using GraphPad Prism (version 6.02)

### Microscopy

Cells were plated on glass slides and were treated with compound at the indicated concentrations for 18h before imaging. Digital images were taken using a Veleta camera (Olympus Soft Imaging Solutions).

### Acid Phosphatase (APH) assay

The APH assay, which measures the activity of acidic phosphatase in the cells, was optimized for 3D cultures as follows. Only 70µl of medium was removed in order not to suck up the spheres. Then 60µl of PBS was added per well and 100µl of APH buffer (0.1 M sodium acetate pH 5.2 and 0.1 % triton X-100) containing the substrate (2mg/ml p-nitrophenyl phosphate, final pH 4.8 from Thermo Scientific). After 2 hours incubation for the 2D culture and 5 hours for the 3D culture, 10µl of 1 M NaOH was added to stop the APH activity. Absorbance at 405nm was measured within 10 minutes using a FluoStar OPTIMA plate reader (BMG Labtech). Viability rate was calculated as per cent of CTR untreated cells. All data are expressed as the mean ± SD of at least 8 replicates.

### Long term toxicity

SK-Mel-28 and Panc-1 cells were plated on 96 well plates. Three hours post-seeding, cells were treated with 10 µM, 1 µM, 0.1 µM and 0.01 µM of **S2** or vehicle (DMSO). Viability assay was conducted after 24 h, 72 h, 5 days, 10 days, 15 days, 20 days and 25 days, as described above. Media was changed every two days. Cells (treated and controls) were split at 80% confluence, as follows: each round of split resulted in two kinds of plates for each cell line: 1: 96-well black clear-bottom microtiter for viability test with 3000 cells/well, and 2: 48 well plate for continuous growing with 6000 cells/well. Cell viability at each concentration was quantified relative to DMSO control at any time point (each time point had its own control), and was presented as fraction of control. Each treatment and the adequate control and was performed in 3, 4 and 6 repeats. Error bars indicate standard error (SE).

### Results

*Effect of S1 on Panc-1 cells in 2D and 3D culture cell viability (APH assay)* Treatment with **S1** in Panc-1 cells in 2D culture, led to loss of viability at 30 µM in a time course manner (after 24h ,48h and 72h a decrease of 43%, 63% and 70 %, respectively, in of cell viability was evident). Treatment with **S1** in Panc-1 cells in 3D culture, did not affect cells after 24h, however, after 48h and 72h a decrease of 55% and 64% in cell viability, was evident. At this concentration vacuoles were formed (Data not shown). Triptolide was used as positive control at (40 µM). Results are presented in Figures 1 a (2D) and 1 b (3D).

### Effect of S1 on HPAF II, and Panc-1 3D culture cell viability (automatic drug screen, ATP viability assay)

In order to confirm the results presented in figure 1, **S1** was further tested in 2 different cell lines in 3D culture format, using an automatic drug screening assay. The effect of Vacquinol-1 RS **(S1)** on cell viability was measured after 72h using the CellTiter-Glo assay (Promega) adapted for 3D culture.

HPAF II cells, which exhibit a mutation both in KRAS and P53 genes, showed a marked decrease in viability at 30-10 µM, compared to control. Panc-1 cells, which exhibit a mutation in the Kras and P53 gene showed a marked decrease in viability at 30 and 25 µM and ∼20% decrease in viability at lower concentrations (20-10 µM), compared to control.

KPC cells, which are mouse origin showed a complete loss of viability at 30-15 µM, and ∼50% reduction in viability at 10 µM, compared to control. Results are presented in figure 2.

### Effect of S1 on BxPCl-3, MIA PaCa2 and U3013 cells cell viability (ATP viability assay)

MIA-PaCa-2 cell line which carries RAS mutation, and BxPCI-2 cell line which exhibit wild type RAS were also tested for the effect of **S1** on their viability, as measured by ATP viability assay. Both cell lines showed an IC₅₀ ranging between 12-19 µM at 24h, 48h and 72h, which further supports an effect of **S1** on viability of pancreatic adenocarcinoma cell lines, at a similar range of concentrations. As positive control glioblastoma cell line U3013 was tested in parallel for the effect of Vacquinol-1 RS **(S1)** on cell viability. The IC₅₀ ranged 3.5-1.9 µM. Results are presented in Figure 3.

### Effect of S2 on SK-Mel-28 cell viability (ATP viability assay)

Human malignant melanoma cell line SK-Mel-28 was tested for its sensitivity to **S2** treatment at various concentrations for 24, 48 and 72 hours. Vacquinol-1 SR **(S2)** treatment resulted in IC₅₀ of 3.6, 2.4 and 2.1 µM respectively, suggesting a high toxicity **of S2** in this cell line. Results are presented in Figure 4.

### Induction of vacuolization by S2 in SK-Mel-28, Panc-1 and KPC cell lines

**S2** was shown to stimulate vacuole formation. Bright field images of cells treated as follows, are presented in figure 5 as follows: SK-Mel-28 cells treated with DMSO (A1), 5µM **S2** (A2) or 10µM **S2** (A3) for 18h. Panc-1 cells treated with DMSO (B1), 5µM **S2** (B2) or 10µM **S2** (B3) for 18h. KPC cells treated with with DMSO (C1), 5µM **S2** (C2) or 10µM **S2** (C3) for 18h. Note the lack of vacuoles in vehicle treated cells (A1, B1, C1).

### Long term toxicity of S2 in SK-Mel-28 and Panc-1 cells

SK-Mel-28 and Panc-1 cells were plated on 96 well plates. Three hours post-seeding, cells were treated with 10 µM, 1 µM, 0.1 µM and 0.01 µM of Vacquionol-1 SR **(S2)** or vehicle (DMSO). Viability assay was conducted after 24 h, 72 h, 5 days, 10 days, 15 days, 20 days and 25 days, as described above. Media was changed every two days. Cells (treated and controls) were split at 80% confluence, as follows: each round of split resulted in two kinds of plates for each cell line: 1: 96-well black clear-bottom microtiter for viability test with 3000 cells/well, and 2: 48 well plate for continuous growing with 6000 cells/well. Cell viability at each concentration was quantified relative to DMSO control at any time point (each time point had its own control), and was presented as fraction of control. Each treatment and the adequate control and was performed in 3, 4 and 6 repeats. Error bars indicate standard error (SE). Results are presented in Figure 6.

### Example 5.

### Ex vivo assessment of S1 compound using a 3D clonogenic assay

The anti-tumor efficacy of **S1** in the patient-derived tumor xenograft (PDX) tumor panel was assessed, where the efficacy of **S1** in PDX tumor panel in the 96-well format, in 10 concentrations in duplicate was performed. Colony formation of tumor cell suspensions in a soft-agar matrix was observed and quantified. The output is IC₅₀ values and dose response-relationships based on number of colonies in control and treatment wells, as identified by an automated image analysis algorithm. Stock solution of **S1** was prepared in 100% DMSO at 10 mM concentration (stored at -20°C) and reused for each experiment. Compound final test concentrations: Dilution series ½ i.e.: 50µM, 15, 5, 1.5, 0.5, 0.15, 0.05, 0.015, 0.005 and 0.0015. Results are presented in Table 2.

### Materials and methods

### Tumor material

Tumor xenografts (also referred to as patient-derived tumor xenografts) were passaged as subcutaneous xenografts in NMRI nu/nu mice. Tumor xenograft were removed from mice under sterile conditions, mechanically disaggregated and subsequently incubated with an enzyme cocktail consisting of collagenase type IV (41 U/mL), DNase I (125 U/mL), hyaluronidase type III (100 U/mL) and dispase II (1.0 U/mL) in RPMI 1640 medium at 37°C for 60 - 120 minutes. Cells were passed through sieves of 100 µm and 40 µm mesh size and washed with RPMI 1640 medium. The percentage of viable cells was determined in a Neubauer-hemocytometer using trypan blue exclusion. Aliquots of the cells were frozen down, and stored in liquid nitrogen.

### 3D Clonogenic assay, 96 well format

The clonogenic assay test was carried out in 96 well plate format. For each test a frozen aliquot of tumor cells prepared from tumor xenografts was thawed and assay plates were prepared as follows: each test well contained 3 layers of equal volume, 2 layers of semi-solid medium (bottom and top layer), and one layer of medium supernatant, with or without test compound. The bottom layer consisted of 0.05 mL/well Iscove's Modified Dulbecco's Medium (Invitrogen), supplemented with 20% (v/v) fetal calf serum (Sigma), 0.01% (w/v) gentamicin (Invitrogen) and 0.75% (w/v) agar (BD Biosciences). Tumor cells were added to 0.05 mL of the same culture medium supplemented with 0.4% (w/v) agar and plated onto the bottom layer. After 24 h, test compounds were added after serial dilution in DMSO and transfer in cell culture medium, and left on the cells for the duration of the experiment (continuous exposure, 0.05 mL drug overlay). Every dish included six untreated control wells and drug-treated groups in duplicate at 10 concentrations. Cultures were incubated at 37 °C and 7.5% CO₂ in a humidified atmosphere for 8 to 13 days and monitored closely for colony growth using an inverted microscope. Within this period, *ex vivo* tumor growth lead to the formation of colonies with a diameter of >50 µm. At the time of maximum colony formation, counts were performed with an automatic image analysis system. 24 h prior to evaluation, vital colonies were stained with a sterile aqueous solution of 2-(4-iodophenyl)-3-(4-nitrophenyl)-5-phenyltetrazolium chloride (1 mg/mL, 100 µL/well). Sigmoidal concentration-response curves were fitted to the data points obtained for each tumor model using 4 parameter non-linear curve fit (Oncotest Warehouse Software). IC₅₀ values are reported as relative IC₅₀ values, being the concentration of test compound that give a response (inhibition of colony formation/viability) half way between the top and bottom plateau of the sigmoidal concentration-response curve (inflection point of the curve), or as absolute IC₅₀ values, being the concentration of test compound at the intersection of the concentration-response curves with T/C = 50%. For calculation of mean IC₅₀ values the geometric mean was used. Results are presented as mean graph plots or heat maps (individual IC₅₀ values relative to the geometric mean IC₅₀ value) over all cell lines as tested.

### Results

*Ex vivo* effects of **S1** on different patient-derived 3D tumors were established by quantitative establishment of the dose-response measuring colonies surviving in the presence of increasing concentrations of **S1.** Data are presented in Table 2 below as absolute and relative IC50 values, and T/C values. **S1** inhibited colony formation in a concentration-dependent and selective manner with a relative mean IC50 value of 8.102 µM (absolute mean IC50 value = 8.888 µM). The bottom plateaus of the concentration-effect curves of tested xenografts were in the range from 0% to 47%, with a major proportion < 25%, indicating pronounced inhibition of tumor colony growth. Based on relative IC50 values, above average activity (individual IC50 value < ½ mean IC50) was observed in 2/33 models, namely BXF 1258 (bladder cancer) and LXFA 526 (NSC lung cancer). IC50 value in these sensitive tumors were on average 3-fold lower than the mean of all tumors as tested. However, most tumors tested (eg. urothelial carcinoma BXF439 metastasis, BXF1036 metastasis, BXF1218 metastasis BXF1258 recurrent; metastatic colorectal adenocarcinoma CXF1103, CXF1297, CXF280, CXF94, CXF975; gastric adenocarcinoma GXF1172 metastasis, GXF214 primary, GXF251 primary, GXF97 primary; head and neck squamous cell carcinoma HNXF908 primary, HNXF1842 primary; lung adenocarcinoma LXFA1041 metastasis, LXFA1584 primary, LXFA1647 metastasis, LXFA297 metastasis, LXFA526 metastasis, LXFA623 metastasis, LXFA629 primary, LXFA923 metastasis; lung large cell carcinoma LXFL1121 primary, LXFL1674 primary, LXFL430 primary, LXFL529 primary) displayed an IC₅₀ in the range 2-15 µM, thus, well in within plasma exposures that can be obtained based on pharmacokinetic data. Only three tumors displayed IC₅₀ above 15 µM (BXF1352, CXF647, LXFA289). The relative IC₅₀ is determined as the concentration that gives a response half way between the maximal signal (top plateau) and the maximally inhibited signal (bottom plateau) (= inflection point of the sigmoidal concentration effect curve). The absolute IC₅₀ is determined as the concentration at the intersection of the concentration effect curve with T/C = 50%. If T/C<50% (T/C>75%) was detected for all test concentrations, the lowest (highest) concentration is given and used for calculation of the mean IC₅₀ value.Table 2 shows the IC₅₀ of *ex vivo* patient-derived tumor xenograft (PDX) from various tumors.

**Table 2.**

| | **30060 uM** | | | **30060 uM** | | |
|---|---|---|---|---|---|---|
| **Tumor model** | **rel**. **IC₅₀ (µM)** | **t** | **b** | **abs. IC₅₀ (µM)** | **t** | **b** |
| BXF 1036 | 4.997 | 95 | 30 | 5.302 | 95 | 30 |
| BXF 1218 | 6.019 | 100 | 15 | 6.163 | 100 | 15 |
| BXF 1228 | 6.666 | 98 | 22 | 8.116 | 98 | 22 |
| BXF 1258 | 2.541 | 103 | 44 | 7.473 | 103 | 44 |
| BXF 1352 | 18.978 | 97 | 0 | 18.507 | 97 | 0 |
| BXF 439 | 12.771 | 95 | 34 | 13.997 | 95 | 34 |
| CXF 1103 | 6.472 | 99 | 47 | 11.509 | 99 | 47 |
| CXF 1297 | 6.369 | 90 | 15 | 6.415 | 90 | 15 |
| CXF 1729 | 8.816 | 104 | 8 | 8.872 | 104 | 8 |
| CXF 1783 | 8.811 | 100 | 42 | 9.257 | 100 | 42 |
| CXF 2025 | 8.613 | 97 | 33 | 8.866 | 97 | 33 |
| CXF 243 | 8.634 | 102 | 46 | 9.224 | 102 | 46 |
| CXF 280 | 8.622 | 101 | 5 | 8.646 | 101 | 5 |
| CXF 647 | 16.491 | 97 | 20 | 24.156 | 97 | 20 |
| CXF 676 | 8.784 | 105 | 19 | 8.919 | 105 | 19 |
| CXF 742 | 41.260 | 100 | 0 | 41.251 | 100 | 0 |
| CXF 94 | 12.663 | 98 | 8 | 13.314 | 98 | 8 |
| CXF 975 | 8.618 | 105 | 19 | 8.746 | 105 | 19 |
| GXA 3023 | 5.019 | 90 | 30 | 5.260 | 90 | 30 |
| GXA 3040 | 8.789 | 94 | 13 | 8.835 | 94 | 13 |
| GXA 3044 | 8.946 | 101 | 15 | 9.084 | 101 | 15 |
| GXF 1172 | 12.472 | 79 | 28 | 12.778 | 79 | 28 |
| GXF 214 | 4.892 | 99 | 6 | 4.918 | 99 | 6 |
| GXF 251 | 5.256 | 111 | 34 | 6.024 | 111 | 34 |
| GXF 97 | 5.710 | 97 | 20 | 6.214 | 97 | 20 |
| HNXF 1842 | 5.530 | 77 | 15 | 5.399 | 77 | 15 |
| HNXF 1853 | 8.773 | 106 | 50 | 50.000 | 106 | 50 |
| HNXF 536 | 7.467 | 99 | 14 | 8.208 | 99 | 14 |
| HNXF 908 | 5.121 | 90 | 17 | 5.194 | 90 | 17 |
| LXFA 1041 | 5.246 | 95 | 10 | 5.302 | 95 | 10 |
| LXFA 1584 | 6.792 | 95 | 31 | 7.810 | 95 | 31 |
| LXFA 1647 | 11.948 | 98 | 13 | 12.141 | 98 | 13 |
| LXFA 289 | 39.339 | 94 | 21 | 45.433 | 94 | 21 |
| LXFA 297 | 8.446 | 100 | 24 | 8.583 | 100 | 24 |
| LXFA 526 | 2.912 | 96 | 10 | 3.234 | 96 | 10 |
| LXFA 623 | 4.616 | 92 | 10 | 4.627 | 92 | 10 |
| LXFA 629 | 8.848 | 108 | 26 | 9.094 | 108 | 26 |
| LXFA 923 | 8.436 | 101 | 21 | 8.555 | 101 | 21 |
| LXFA 983 | 4.596 | 96 | 30 | 4.814 | 96 | 30 |
| LXFE 397 | 4.771 | 100 | 35 | 5.139 | 100 | 35 |
| LXFL 1072 | 7.578 | 101 | 0 | 7.601 | 101 | 0 |
| LXFL 1121 | 8.612 | 101 | 9 | 8.663 | 101 | 9 |
| LXFL 1674 | 6.594 | 93 | 9 | 6.684 | 93 | 9 |
| LXFL 430 | 8.984 | 99 | 14 | 9.060 | 99 | 14 |
| LXFL 529 | 10.076 | 92 | 11 | 10.443 | 92 | 11 |
| MAXF 1162 | 4.628 | 90 | 12 | 4.644 | 90 | 12 |
| MAXF 1322 | 3.821 | 93 | 21 | 4.766 | 93 | 21 |
| MAXF 574 | 50.000 | n.d. | n.d. | 50.000 | n.d. | n.d. |
| MAXF 713 | 8.598 | 108 | 43 | 9.163 | 108 | 43 |
| MEXF 1765 | 8.528 | 84 | 23 | 8.579 | 84 | 23 |
| MEXF 1792 | 6.148 | 99 | 5 | 6.190 | 99 | 5 |
| MEXF 274 | 8.686 | 103 | 17 | 8.844 | 103 | 17 |
| MEXF 276 | 4.654 | 95 | 15 | 4.732 | 95 | 15 |
| MEXF 622 | 9.138 | 86 | 21 | 9.212 | 86 | 21 |
| MEXF 672 | 8.562 | 96 | 39 | 8.960 | 96 | 39 |
| OVXF 1023 | 6.838 | 100 | 11 | 7.491 | 100 | 11 |
| OVXF 1353 | 4.724 | 101 | 23 | 4.909 | 101 | 23 |
| OVXF 1544 | 12.830 | 107 | 22 | 13.514 | 107 | 22 |
| OVXF 899 | 6.157 | 100 | 18 | 6.377 | 100 | 18 |
| PAXF 1872 | 8.892 | 89 | 42 | 9.299 | 89 | 42 |
| PAXF 1900 | 12.218 | 114 | 33 | 13.153 | 114 | 33 |
| PAXF 1998 | 8.540 | 104 | 38 | 8.890 | 104 | 38 |
| PAXF 2005 | 9.061 | 106 | 11 | 9.160 | 106 | 11 |
| PAXF 2033 | 8.891 | 110 | 27 | 9.185 | 110 | 27 |
| PAXF 2045 | 7.033 | 95 | 22 | 8.141 | 95 | 22 |
| PAXF 2046 | 9.454 | 100 | 38 | 18.569 | 100 | 38 |
| PAXF 2053 | 5.670 | 101 | 18 | 5.873 | 101 | 18 |
| PAXF 2059 | 12.961 | 130 | 18 | 13.825 | 130 | 18 |
| PAXF 2094 | 8.641 | 105 | 30 | 8.875 | 105 | 30 |
| PAXF 2116 | 8.847 | 112 | 32 | 9.160 | 112 | 32 |
| PAXF 546 | 9.073 | 96 | 25 | 9.225 | 96 | 25 |
| PRXF MRI-H-1579 | 8.763 | 112 | 21 | 8.943 | 112 | 21 |
| PRXF PC-3M | 16.884 | 98 | 3 | 16.933 | 98 | 3 |
| PXF 1752 | 8.854 | 109 | 15 | 8.982 | 109 | 15 |
| PXF 537 | 8.634 | 80 | 24 | 8.668 | 80 | 24 |
| RXF 1114 | 7.979 | 94 | 30 | 8.176 | 94 | 30 |
| RXF 1183 | 50.000 | n.d. | n.d. | 50.000 | n.d. | n.d. |
| RXF 1220 | 43.354 | 99 | 0 | 43.292 | 99 | 0 |
| RXF 1781 | 11.060 | 112 | 8 | 11.125 | 112 | 8 |
| RXF 486 | 12.732 | 97 | 5 | 12.774 | 97 | 5 |
| RXF 616 | 8.767 | 89 | 17 | 8.808 | 89 | 17 |
| RXF 631 | 28.104 | 91 | 0 | 24.577 | 91 | 0 |
| SXFS 117 | 8.582 | 91 | 4 | 8.552 | 91 | 4 |
| SXFS 1937 | 9.902 | 120 | 18 | 10.067 | 120 | 18 |
| SXFS 627 | 5.739 | 96 | 7 | 5.840 | 96 | 7 |
| Mean IC₅₀ (qeometr.) | 8.700 | | | 9.470 | | |

| | | | | | | |
|---|---|---|---|---|---|---|
| Abbreviations: BXF - Bladder; CXF - Colorectal; GXA - Asian Gastric; GXF - Caucasian Gastric; HNXF - Caucasian Head and Neck; LXF - Lung A Adeno, E - Epidermoid, L - Large Cell; MAXF - Breast; MEXF - Melanoma; OVXF-Ovarian; PAXF - Pancreas; PRXF - Prostate; PXF - Pleuramesothelioma; RXF - Renal; SXF - Sarcoma; O - Osteosarcoma; S - Soft Tissue. t,b: top, bottom plateau of concentration-effect curve. n.d.: not determined. | | | | | | |

### Items of the invention

Below follows a number of non-limiting items of the invention.
1. (S)-(2-(4-chlorophenyl)quinolin-4-yl)((R)-piperidin-2-yl)methanol, or a pharmaceutically acceptable salt or solvate thereof, for use in the treatment of systemic cancer.
2. (S)-(2-(4-chlorophenyl)quinolin-4-yl)((R)-piperidin-2-yl)methanol, for use according to item 1, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.
3. (S)-(2-(4-chlorophenyl)quinolin-4-yl)((R)-piperidin-2-yl)methanol, for use according to item 1 or 2, wherein said systemic cancer is pancreatic cancer.
4. (S)-(2-(4-chlorophenyl)quinolin-4-yl)((R)-piperidin-2-yl)methanol, for use according to item 1 or 2, wherein said systemic cancer is skin cancer.
5. (S)-(2-(4-chlorophenyl)quinolin-4-yl)((R)-piperidin-2-yl)methanol, for use according to any one of items 1 to 4, wherein side effects on the central nervous system are minimized.
6. (R)-(2-(4-chlorophenyl)quinolin-4-yl)((S)-piperidin-2-yl)methanol, or a pharmaceutically acceptable salt or solvate thereof, for use in the treatment of systemic cancer.
7. (R)-(2-(4-chlorophenyl)quinolin-4-yl)((S)-piperidin-2-yl)methanol, for use according to item 6, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.
8. (R)-(2-(4-chlorophenyl)quinolin-4-yl)((S)-piperidin-2-yl)methanol, for use according to item 6 or 7, wherein said systemic cancer is pancreatic cancer.
9. (R)-(2-(4-chlorophenyl)quinolin-4-yl)((S)-piperidin-2-yl)methanol, for use according to item 6 or 7, wherein said systemic cancer is skin cancer.
10. A method of treating systemic cancer, comprising administering to a subject a therapeutically effective amount of (S)-(2-(4-chlorophenyl)quinolin-4-yl)((R)-piperidin-2-yl)methanol or a pharmaceutically acceptable salt or solvate thereof.
11. The method according to item 10, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.
12. The method according to item 10, wherein said systemic cancer is pancreatic cancer.
13. The method according to item 10, wherein said systemic cancer is skin cancer.
14. The method according to item 10, wherein side effects on central nervous system are minimized.
15. A method of treating systemic cancer, comprising administering to a subject a therapeutically effective amount of (R)-(2-(4-chlorophenyl)quinolin-4-yl)((S)-piperidin-2-yl)methanol or a pharmaceutically acceptable salt or solvate thereof.
16. The method according to item 15, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.
17. The method according to item 15, wherein said systemic cancer is pancreatic cancer.
18. The method according to item 15, wherein said systemic cancer is skin cancer.
19. The method according to item 15, wherein side effects on central nervous system are minimized.
20. Use of (S)-(2-(4-chlorophenyl)quinolin-4-yl)((R)-piperidin-2-yl)methanol or a pharmaceutically acceptable salt or solvate thereof, in the manufacture of a medicament for treatment of systemic cancer.
21. The use according to item 20, wherein said wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.
22. The use according to item 20, wherein said systemic cancer is pancreatic cancer.
23. The use according to item 20, wherein said systemic cancer is skin cancer.
24. The use according to any one of items 20 to 23, wherein side effects on the central nervous system are minimized.
25. Use of (R)-(2-(4-chlorophenyl)quinolin-4-yl)((S)-piperidin-2-yl)methanol or a pharmaceutically acceptable salt or solvate thereof, in the manufacture of a medicament for treatment of systemic cancer.
26. The use according to item 25, wherein said wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.
27. The use according to item 25, wherein said systemic cancer is pancreatic cancer.
28. The use according to item 25, wherein said systemic cancer is skin cancer.
29. The use according to any one of items 25 to 28, wherein side effects on central nervous system are minimized.
30. A phamaceutical composition comprising a mixture of (S)-(2-(4-chlorophenyl)quinolin-4-yl)((R)-piperidin-2-yl)methanol, or a pharmaceutically acceptable salt or solvate thereof; and (R)-(2-(4-chlorophenyl)quinolin-4-yl)((S)-piperidin-2-yl)methanol, or a pharmaceutically acceptable salt or solvate thereof, together with pharmaceutically acceptable excipients and diluents, for use in the treatment of systemic cancer.
31. The composition for use according to item 30, wherein said mixture comprises at least 90% of (S)-(2-(4-chlorophenyl)quinolin-4-yl)((R)-piperidin-2-yl)methanol.
32. The composition for use according to item 30, wherein said mixture comprises at least 95% of (S)-(2-(4-chlorophenyl)quinolin-4-yl)((R)-piperidin-2-yl)methanol.
33. The composition for use according to item 30, wherein said mixture comprises at least 99% of (S)-(2-(4-chlorophenyl)quinolin-4-yl)((R)-piperidin-2-yl)methanol.
34. The composition for use according to any one of items 30 to 33, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, skin, hematological/lymphoid, pleura mesothelioma, renal, sarcoma, soft tissue, testicular, uterine body cancer.
35. The composition for use according to any one of items 30 to 33, wherein said systemic cancer is pancreatic cancer.
36. The composition for use according to any one of items 30 to 33, wherein said systemic cancer is skin cancer.
37. The composition for use according to any one of items 30 to 36, wherein side effects on central nervous system are minimized.
38. (S)-(2-(4-chlorophenyl)quinolin-4-yl)((R)-piperidin-2-yl)methanol, or a pharmaceutically acceptable salt or solvate thereof.
39. A method of stereoselective synthesis method of (R)-(2-(4-chlorophenyl)quinolin-4-yl)((S)-piperidin-2-yl)methanol, as set out in example 1.
40. A method of stereoselective synthesis method of (S)-(2-(4-chlorophenyl)quinolin-4-yl)((R)-piperidin-2-yl)methanol, as set out in example 2.

## Claims

1. (S)-(2-(4-chlorophenyl)quinolin-4-yl)((R)-piperidin-2-yl)methanol, or a pharmaceutically acceptable salt or solvate thereof, for use in the treatment of systemic cancer.

2. (S)-(2-(4-chlorophenyl)quinolin-4-yl)((R)-piperidin-2-yl)methanol, for use according to claim 1, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

3. (S)-(2-(4-chlorophenyl)quinolin-4-yl)((R)-piperidin-2-yl)methanol, for use according to claim 1 or 2, wherein said systemic cancer is pancreatic cancer.

4. (S)-(2-(4-chlorophenyl)quinolin-4-yl)((R)-piperidin-2-yl)methanol, for use according to claim 1 or 2, wherein said systemic cancer is skin cancer.

5. (S)-(2-(4-chlorophenyl)quinolin-4-yl)((R)-piperidin-2-yl)methanol, for use according to any one of claims 1 to 4, wherein side effects on the central nervous system are minimized.

6. (R)-(2-(4-chlorophenyl)quinolin-4-yl)((S)-piperidin-2-yl)methanol, or a pharmaceutically acceptable salt or solvate thereof, for use in the treatment of systemic cancer.

7. (R)-(2-(4-chlorophenyl)quinolin-4-yl)((S)-piperidin-2-yl)methanol, for use according to claim 6, wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

8. (R)-(2-(4-chlorophenyl)quinolin-4-yl)((S)-piperidin-2-yl)methanol, for use according to claim 6 or 7, wherein said systemic cancer is pancreatic cancer.

9. (R)-(2-(4-chlorophenyl)quinolin-4-yl)((S)-piperidin-2-yl)methanol, for use according to claim 6 or 7, wherein said systemic cancer is skin cancer.

10. A phamaceutical composition comprising a mixture of (S)-(2-(4-chlorophenyl)quinolin-4-yl)((R)-piperidin-2-yl)methanol, or a pharmaceutically acceptable salt or solvate thereof; and (R)-(2-(4-chlorophenyl)quinolin-4-yl)((S)-piperidin-2-yl)methanol, or a pharmaceutically acceptable salt or solvate thereof, together with pharmaceutically acceptable excipients and diluents, for use in the treatment of systemic cancer.

11. The composition for use according to claim 10, wherein said mixture comprises at least 90% of (S)-(2-(4-chlorophenyl)quinolin-4-yl)((R)-piperidin-2-yl)methanol.

12. The composition for use according to claim 10, wherein said mixture comprises at least 95% of (S)-(2-(4-chlorophenyl)quinolin-4-yl)((R)-piperidin-2-yl)methanol.

13. The composition for use according to claim 10, wherein said mixture comprises at least 99% of (S)-(2-(4-chlorophenyl)quinolin-4-yl)((R)-piperidin-2-yl)methanol.

14. The composition for use according to any one of claims 10 to 13,
wherein said systemic cancer is selected from pancreatic, lung, thyroid,urinary tract, bladder, hematopoietic, anal, colorectal, gastrointestinal, gastric, head and neck, liver, melanoma, multiple myeoloma, neuroblastoma, oesophagal, ovarian, prostate, salivary, intestinal, skin, hematological/lymphoid, cervical, pancreatic, hematological/lymphoid, pleura mesothelioma, renal, sarcoma, soft tissue, testicular, uterine body cancer.

15. (S)-(2-(4-chlorophenyl)quinolin-4-yl)((R)-piperidin-2-yl)methanol, or a pharmaceutically acceptable salt or solvate thereof.
